(19) Europäisches Patentamt
European Patent Office
Office européen des brevets

(11) EP 4 692 050 A1

(12) **EUROPEAN PATENT APPLICATION**
published in accordance with Art. 153(4) EPC

(43) Date of publication:
**11.02.2026 Bulletin 2026/07**

(21) Application number: **24780938.7**

(22) Date of filing: **01.04.2024**

(51) International Patent Classification (IPC):
***C07C 271/44*** *(2006.01)* ***C07C 275/32*** *(2006.01)*
***C07D 295/205*** *(2006.01)*

(52) Cooperative Patent Classification (CPC):
**C07C 271/44; C07C 275/32; C07D 295/205**

(86) International application number:
**PCT/JP2024/013473**

(87) International publication number:
**WO 2024/204846 (03.10.2024 Gazette 2024/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **31.03.2023 JP 2023058757**

(71) Applicant: **Mitsui Chemicals, Inc.**
**Tokyo 104-0028 (JP)**

(72) Inventors:
- **ISOGAI Makoto**
  **Sodegaura-shi, Chiba 299-0265 (JP)**
- **TAKANO Shotaro**
  **Sodegaura-shi, Chiba 299-0265 (JP)**
- **YAMADA Wataru**
  **Sodegaura-shi, Chiba 299-0265 (JP)**
- **KIMURA Takashi**
  **Kuga-gun, Yamaguchi 740-0061 (JP)**

(74) Representative: **J A Kemp LLP**
**80 Turnmill Street**
**London EC1M 5QU (GB)**

(54) **CARBAMATE COMPOUND**

(57) A carbamate compound represented by the following formula (0).

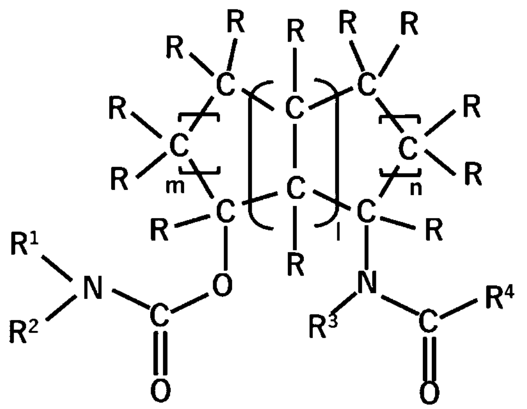

(0)

wherein l is 0 or 1, m is an integer of 1 to 4, n is an integer of 1 to 4, $R^1$ and $R^2$ are substituents having a "$R^{10}$-$CR_2$-" structure, $R^3$ is a hydrogen atom or a substituent having a "$R^{10}$-$CR_2$-" structure, $R^4$ is a substituent selected from a substituent having a "$R^{10}$-$CR_2$-" structure, a substituent having a "$R^{10}$-$At^{16}$-" structure, and a substituent having a "$R^{10}_2$-$At^{15}$-" structure, $At^{15}$ is a Group 15 atom, $At^{16}$ is a Group 16 atom, R and $R^{10}$ are each a group containing an atom selected from carbon, hydrogen, and Groups 15, 16, and 17 elements, which has 0 to 17 carbon atoms and 0 to 4 heteroatoms, $R^1$ to $R^4$ and R may bond to each other to form a monocyclic or polycyclic ring, and R and $R^{10}$ may bond to each other to form a monocyclic ring, a polycyclic ring, or a multiple bond.



Processed by Luminess, 75001 PARIS (FR)

## Description

### Technical Field

The present invention relates to a carbamate compound.

### Background Art

Carbamate compounds are known to be in use in applications of not only intermediates of chemicals, such as solvents, medicines, and pesticides, but also, for example, nylon raw materials. It has been also reported that carbamate compounds are used as a Mg compound-supported titanium catalysts that are used in olefin polymerization.

A catalyst for olefin polymerization is one of the techniques greatly developed up to the present, which was triggered by the discovery of so-called Ziegler-Natta catalysts, for which Ziegler reported in 1953 that ethylene was polymerized even at low pressures by use of a combination of titanium tetrachloride and an organoaluminum compound and Natta subsequently reported the first propylene polymerization by use of a combination of titanium trichloride and a halogen-containing organoaluminum compound. Meanwhile, it has been found that catalysts containing titanium tetrachloride, a magnesium compound, and a Lewis base, which are referred to as third generation catalysts, can achieve both high polymerization activity (high productivity) and high stereoregularity in propylene polymerization. This provided one opportunity to allow propylene polymers (polypropylene) to spread around the world.

A Lewis base (hereinafter, also referred to as an "internal donor"), one of major components of the above third generation catalyst component (hereinafter, also referred to as a "solid titanium catalyst component"), was found to greatly affect catalyst performance, and various Lewis bases have been developed so far.

As Lewis bases for use in Ziegler-Natta catalysts, ethyl benzoate, phthalic esters, 1,3-diketone (Patent Literature 1), malonic ester (Patent Literature 2), succinic ester (Patent Literature 3), 2,4-pentanediol diester (Patent Literature 4), naphthalenediol diester (Patent Literature 5), and catechol diester (Patent Literature 6), for example, have been reported. Mainly enterprises vigorously make research and development in this field even today. It has been also reported that a carbamate compound having a specific structure is suitable (Patent Literature 7 to 9).

### Citation List

#### Patent Literature

Patent Literature 1: JP 2005-226076A
Patent Literature 2: JP 2000-516987A
Patent Literature 3: JP 2002-542347A
Patent Literature 4: JP 2005-517746A
Patent Literature 5: JP 2011-529888A
Patent Literature 6: JP 2014-500390A
Patent Literature 7: WO2016/184884
Patent Literature 8: US Patent No. 10005859
Patent Literature 9: US Patent No. 10836847


### Summary of Invention

#### Technical Problem

Propylene polymers, while having heat resistance and rigidity similar to those of general-purpose engineering plastics, have an advantage of generating a smaller amount of toxic gas even when combustion-treated, because of being constituted substantially only by carbon and hydrogen.

With recent advances in molding techniques, use of a propylene polymer having higher stereoregularity than before has a possibility of developing higher physical properties (such as rigidity and heat resistance). For this reason, the market has required propylene polymers having higher stereoregularity. From the viewpoint of resource saving and environmental protection, methods for producing a propylene polymer at a high productivity also have been required.

It is thus an object of the present invention to provide an internal donor component suitable for a solid titanium catalyst component capable of producing a propylene polymer having extremely high stereoregularity at a high productivity (high activity), when used mainly for solid titanium catalyst component.

Solution to Problem

As a result of diligent study to solve the above problems, the present inventors have found that a carbamate compound having a specific structure is suitable as, for example, a Lewis base for a solid titanium catalyst component, and completed the present invention. Examples of the present invention will be shown below.

[1] A carbamate compound represented by the following formula (0):

[Chem. 1]

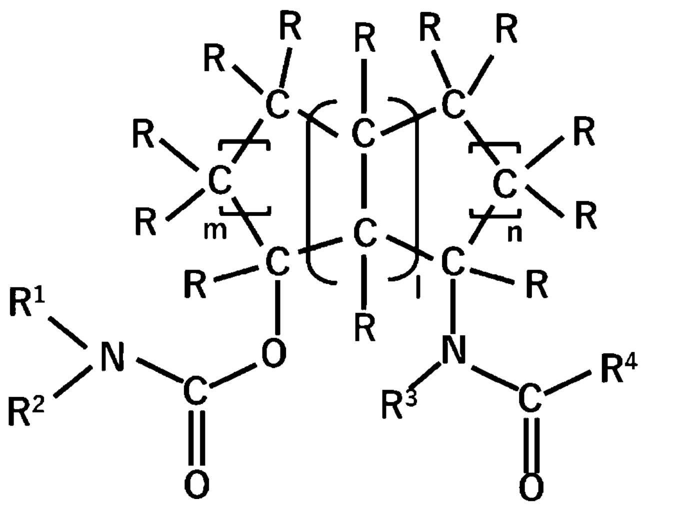

(0)

wherein

l is 0 or 1,
m is an integer of 1 to 4,
n is an integer of 1 to 4,
$R^1$ and $R^2$ are each a substituent having a "$R^{10}$-$CR_2$-" structure,
$R^3$ is a hydrogen atom or a substituent having a "$R^{10}$-$CR_2$-" structure,
$R^4$ is a substituent selected from a substituent having a "$R^{10}$-$CR_2$-" structure, a substituent having a "$R^{10}$-$At^{16}$-" structure, and a substituent having a "$R^{10}_2$-$At^{15}$-" structure,
$At^{15}$ is a Group 15 atom in the periodic table, and $At^{16}$ is a Group 16 atom in the periodic table,
R and $R^{10}$ are each a group containing an atom selected from carbon, hydrogen, and elements in Groups 15, 16, and 17 of the periodic table, which has 0 to 17 carbon atoms and 0 to 4 atoms of the elements in Groups 15, 16, and 17 of the periodic table,
$R^1$ to $R^4$ and R may bond to each other to form a monocyclic or polycyclic ring, and
a plurality of R and $R^{10}$ may bond to each other to form a monocyclic or polycyclic ring or form a multiple bond.

[2] The carbamate compound according to [1], wherein the compound is represented by the following formula (1):

[Chem. 2]

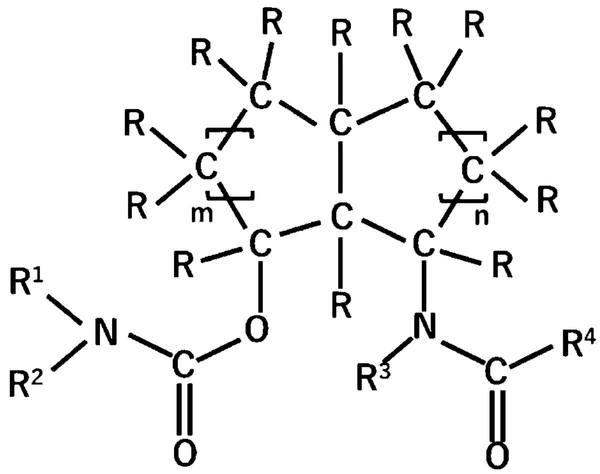

(1)

wherein n is an integer of 2 to 4, and m, $R^1$, $R^2$, $R^3$, $R^4$, and R have the same meaning as m, $R^1$, $R^2$, $R^3$, $R^4$, and R in the formula (0).

[3] The carbamate compound according to [1] or [2], wherein the $R^3$ is a substituent having a "$R^{10}$-$CR_2$-" structure.

[4] The carbamate compound according to any one of [1] to [3], wherein the $R^4$ is a substituent having a "$R^{10}$-$CR_2$-" structure.

[5] The carbamate compound according to any one of [1] to [4], wherein the $At^{15}$ is a nitrogen atom.

[6] The carbamate compound according to any one of [1] to [5], wherein the $At^{16}$ is an oxygen atom.

Advantageous Effects of Invention

The carbamate compound of the present invention can be used as, for example, a pharmaceutical, such as an insecticide, or an intermediate thereof, a raw material of a filler of an optical resolution column, or a Ziegler-Natta catalyst raw material.

Description of Embodiments

[Carbamate compound]

A carbamate compound of the present invention is represented by the following formula (0).

[Chem. 3]

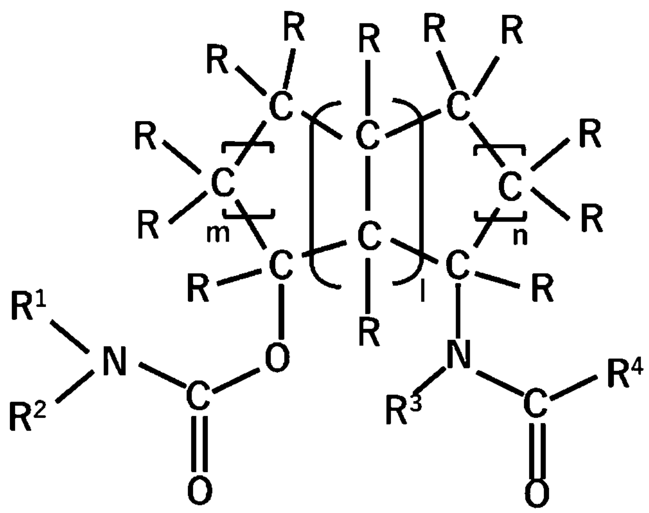

(0)

wherein

l is 0 or 1,
m is an integer of 1 to 4,
n is an integer of 1 to 4,
$R^1$ and $R^2$ are each a substituent having a "$R^{10}$-$CR_2$-" structure,
$R^3$ is a hydrogen atom or a substituent having a "$R^{10}$-$CR_2$-" structure,
$R^4$ is a substituent selected from a substituent having a "$R^{10}$-$CR_2$-" structure, a substituent having a "$R^{10}$-$At^{16}$-" structure, and a substituent having a "$R^{10}_2$-$At^{15}$-" structure,
$At^{15}$ is a Group 15 atom in the periodic table, and $At^{16}$ is a Group 16 atom in the periodic table,
R and $R^{10}$ are each a group containing an atom selected from carbon, elements in hydrogen, and Groups 15, 16, and 17 of the periodic table, which has 0 to 17 carbon atoms and 0 to 4 heteroatoms,
$R^1$ to $R^4$ and R may bond to each other to form a monocyclic or polycyclic ring, and
a plurality of R and $R^{10}$ may bond to each other to form a monocyclic or polycyclic ring or form a multiple bond.

One of the preferable aspects of the structural formula is a compound specified by the following formula (1). In this case, n is an integer of 2 to 4. Other symbols have the same meaning as those in the formula (0).

[Chem. 4]

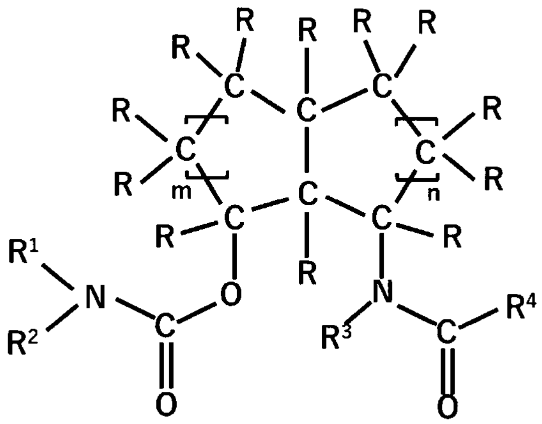

(1)

The $At^{15}$ that is a Group 15 atom in the periodic table is preferably an atom selected from, for example, nitrogen, phosphorus, arsenic, and antimony, more preferably an atom selected from nitrogen and phosphorus, and particularly preferably a nitrogen atom.

The $At^{16}$ that is a Group 16 atom in the periodic table is preferably an atom selected from, for example, oxygen, sulfur, and selenium, more preferably an atom selected from oxygen and sulfur, and particularly preferably an oxygen atom.

The symbol "-" represents a covalent bond, and the term "atom" may refer to the atom itself, or a structure having a covalent bond in a compound or a substituent. For example, in the case of an oxygen atom, the expression "-O-" is referred to as an oxygen atom in some cases.

The R and $R^{10}$ are each a group containing an atom selected from carbon, hydrogen, and elements in Groups 15, 16, and 17 of the periodic table, which has 0 to 17 carbon atoms and 0 to 4 atoms of the elements in Groups 15, 16, and 17 of the periodic table. The Groups 15 and 16 elements can be elements as exemplified in the $At^{15}$ and $At^{16}$. Preferable examples of the Group 17 elements can include fluorine, chlorine, bromine, and iodine, and more preferably an atom selected from fluorine, chlorine, and bromine, still more preferably an atom selected from fluorine and chlorine is still more preferable, and particularly chlorine.

The lower limit value of the number of carbon atoms preferably is one, and the upper limit value thereof is preferably 15, more preferably 13, still more preferably 11, and particularly preferable 9.

The upper limit value of the number of the atoms of the Groups 15, 16, and 17 elements in the periodic table is preferably 3 and more preferably 2.

These substituents ($R^1$, $R^2$, $R^3$, $R^4$, $R^{10}$, and R) are capable of bonding to each other to form, for example, a cyclic structure, a polycyclic structure, or an aromatic structure. In the present invention, unless particularly otherwise described, a double bond or a triple bond is regarded as a two-member ring and a kind of cyclic structure.

The plurality of R and $R^{10}$ can bond to each other to form, for example, a cyclic structure, a polycyclic structure, or

an aromatic structure. When adjacent carbon atoms to which these substituents are bonded form a double bond, these R and $R^{10}$ can be regarded as directly bonding to each other to form the double bond.

The plurality of R and $R^{10}$ may be present, and they have the same structure or a different structure. Specific examples of such substituents include substituted or unsubstituted hydrocarbon groups having 1 to 17 carbon atoms. Examples of such substituents can include aliphatic substituents, alicyclic substituents, and hydrocarbon groups having an aryl group and 6 to 20 carbon atoms. These may be a structure containing any of Groups 15 to 17 atoms in the periodic table (in the present invention, referred to as a heteroatom in some cases) as described above. These are preferably a hydrocarbon group consisting of carbon and hydrogen.

Preferable examples of the hydrocarbon group can include a monovalent hydrocarbon group having 0 to 15 carbon atoms, preferably 0 to 13 carbon atoms, more preferably 1 to 11 carbon atoms, and still more preferably 1 to 9 carbon atoms. In the present invention, when the number of carbon atoms is zero, a hydrogen atom or a covalent bond that forms a double bond is referred to.

Specific examples of such a hydrocarbon group include aliphatic hydrocarbon groups, alicyclic hydrocarbon groups, and aromatic hydrocarbon groups, such as a methyl group, an ethyl group, a n-propyl group, an isopropyl group, a n-butyl group, an isobutyl group, a hexyl group, a heptyl group, an octyl group, a 2-ethylhexyl group, a decyl group, a dodecyl group, a tetradecyl group, a hexadecyl group, an octadecyl group, an eicosyl group, a cyclohexyl group, a substituted or unsubstituted aryl group such as a phenyl group, and a substituted or unsubstituted cycloalkenyl group. Among these, for example, a n-butyl group, an isobutyl group, a hexyl group, an octyl group, and a phenyl group are preferable, and a n-butyl group, an isobutyl group, and a phenyl group are more preferable.

The hydrocarbon group may be a hydrocarbon group containing a heteroatom, such as nitrogen, oxygen, phosphorus, or a halogen, as described above. Such a heteroatom is particularly oxygen or nitrogen. Such substituents can be selected from known structures. More specific preferable examples thereof can include a carbonyl structure-containing group such as a carboxylic acid ester group, an aldehyde group, an acetyl group, or an oxycarbonylalkyl group, an alkoxy group, a substituted or unsubstituted alkoxy group, a substituted or unsubstituted alkenyloxy group, a substituted or unsubstituted cycloalkyloxy group, a substituted or unsubstituted cycloalkenyloxy group, a substituted or unsubstituted aryloxy group, a substituted or unsubstituted heteroaryl group, a substituted or unsubstituted heteroaryloxy group, and a siloxy group.

The $R^1$ and $R^2$ are each a substituent having a "$R^{10}$-$CR_2$-" structure. Examples of such substituents are substantially almost the same as the substituents exemplified as R and $R^{10}$, but a moiety that bonds to nitrogen of a carbamate group described below is limited to carbon. For example, structures that bond to other groups through oxygen or nitrogen, such as an alkoxy group and an amino group, are excluded in such substituents. Specific preferable examples of such substituents include an ethyl group, a propyl group, an isopropyl group, a n-butyl group, an isobutyl group, a hexyl group, an octyl group, and a phenyl group, and more preferably an ethyl group, a propyl group, an isopropyl group, a n-butyl group, an isobutyl group, and a phenyl group, and particularly preferably an ethyl group, a propyl group, an isopropyl group, and a phenyl group.

The $R^3$ is the definition of the $R^1$ or $R^2$ to which a hydrogen atom has been added. The $R^3$ is preferably a substituent having a "$R^{10}$-$CR_2$-" structure. Therefore, specific examples of preferable substituents are also the same as those of the $R^1$ and $R^2$.

The $R^4$ is a substituent selected from a substituent having a "$R^{10}$-$CR_2$-" structure, a substituent having a "$R^{10}$-$At^{16}$-" structure, and a substituent having a "$R^{10}_2$-$At^{15}$-" structure. Specific examples of this $R^4$ are almost the same as the substituents exemplified as the R, $R^{10}$, $R^1$, and $R^2$. The $At^{16}$ is preferably oxygen, and preferable example of the $At^{15}$ is nitrogen. Specific examples of the "$R^{10}$-$At^{16}$-" structure can include an alkoxy group and an aryloxy group, and more specific preferable examples of the substituents can include an ethoxy group, a propoxy group, a butoxy group, an acetyloxy group, an ethylcarbonyloxy group, a metalloyloxy group, a phenoxy group, and a substituted phenoxy group.

Specific examples of "$R^{10}$-$At^{15}$-" structure can include a dimethylamino group, a diethylamino group, a dipropylamino group, a diisopropylamino group, a dibutylamino group, a methylethylamino group, a methylpropylamino group, a diphenylamino group, and a ditolylamino group.

The carbamate compound of the present invention is a structure having a substituent of a carbamate structure and a substituent of an amide structure as shown in the formula (1). Particularly, the substituent having a carbamate structure bonds to A through oxygen (O-), and the substituent of an amide structure bonds to A through nitrogen (N-). In the present invention, the carbamate group and an amide group are simply referred to as a functional group in some cases. The amide group may become a substituent with a different name, such as a carbamate group, due to the structure of $R^4$.

The carbamate compound specified by the formula (0) of the present invention is a structure having a substituent of the carbamate structure and a substituent of an amide structure in a divalent group of such a structure as a formula (0') shown below. The carbamate compound specified by the formula (1) of the present invention is a structure having a substituent of the carbamate structure and a substituent of an amide structure in a divalent group of such a structure as a formula (1') shown below.

[Chem. 5]

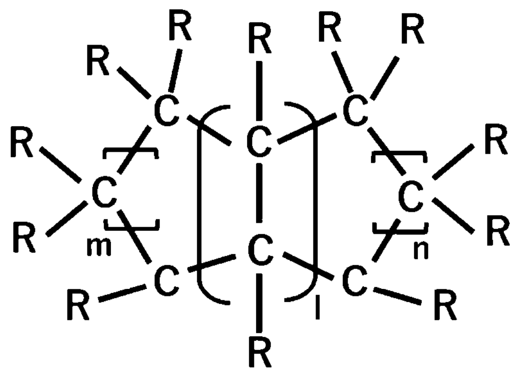

(0')

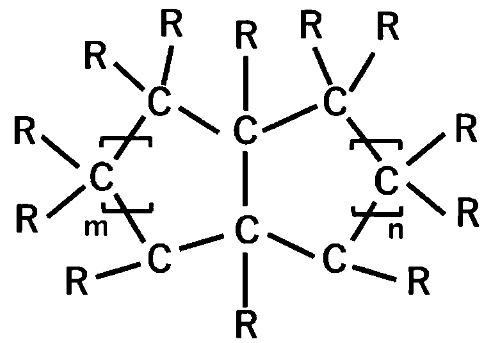

(1')

The symbols such as C and R in the formula (0') and the formula (1') have the same meanings as the symbols in the formula (0) and the formula (1).

l in the formulae (0) and (0') are 0 or 1 and preferably 1.

The molecular skeleton corresponds to the formula (1) in a case where l in the formula (0) is 1 and corresponds to the formula (1') in a case where l in the formula (0') is 1.

m is an integer of 1 to 4. In the case of the formula (0), the value of m is preferably 1 or 2 and more preferably 1. In the case of the formula (1), a preferable lower limit value of m is 2, and a preferable upper limit value is 3.

n is an integer of 1 to 4. In the case of the formula (0), the value of n is preferably 1 or 2 and more preferably 1. In the formula (1), an aspect where the value of n is 1 to 4 is also within the scope of the present invention, but the value of n is preferably 2 to 4, more preferably 2 or 3, and still more preferably 2.

The sum of the m and the n is 2 to 8, preferably 2 to 7, more preferably 2 to 6, and particularly preferably 2 to 5 in the case of the formula (0).

The sum of the m and the n is 3 to 8, preferably 3 to 7, more preferably 3 to 6, and particularly preferably 3 to 5 in the case of the formula (1).

In the case of l = 0 in the formula (0), $R^1$, $R^2$, and $R^4$ are preferably substituents having 2 or more carbon atoms. Particularly, $R^1$, $R^2$, and $R^4$ are preferably hydrocarbon groups consisting of carbon and hydrogen, and more specifically, aliphatic hydrocarbon groups having 2 to 10 carbon atoms, branched aliphatic hydrocarbon groups, and aromatic hydrocarbon groups are preferable.

Among them, $R^1$ and $R^2$ are preferably aliphatic hydrocarbon groups having 2 to 10 carbon atoms, branched aliphatic hydrocarbon groups, and alicyclic hydrocarbon group groups, and the number of carbon atoms is more preferably 2 to 6, still more preferably 2 to 4, and particularly preferably 2 or 3. Specific preferable examples of the substituents can include an ethyl group, a n-propyl group, an isopropyl group, a n-butyl group, an isobutyl group, a 2-butyl group, a pentyl group, a hexyl group, an octyl group, and a decyl group, and more preferably an ethyl group, a propyl group, a butyl group, and a hexyl group, still more preferably an ethyl group and a butyl group, and particularly an ethyl group.

The $R^4$ is more preferably an aromatic hydrocarbon group, still more preferably a phenyl group or a substituted phenyl group, and particularly preferably a phenyl group.

Such cyclic structures as the formula (0') and the formula (1') may be alicyclic structures or aromatic structures. In the case of the alicyclic structure, it is considered that relatively diverse conformations can be formed, and it is thus possible to expect that diverse active species as described below are formed. On the other hand, the aromatic structure can be

considered as a rigid structure in which electronic transfer is likely to occur, and it is thus possible to expect that an effect of electronic negative uniformity as described below becomes significant.

When the alicyclic structure is a structure including a double bond, it seems that R has disappeared at first glance, but it can be considered that R's bonding to adjacent carbon atoms directly bond to each other to form a double bond (which can also be regarded as a two-member ring), and the alicyclic structure is thus a structure within the scope of the present invention. Since the aromatic structure has such a double bond formed at a specific position, the aromatic structure is also a structure within the scope of the present invention.

The carbamate compound of the present invention can be suitably used as an internal donor compound (Lewis base) of a solid titanium catalyst component as described above. The solid titanium catalyst component containing the carbamate compound having such a specific structure tends to have an excellent balance among, for example, activity, stereospecificity, molecular weight controllability, and reaction control. The reason for the solid titanium catalyst component having such an effect is not clear at the moment, but the present inventors presume as described below.

As described above, in the functional groups described above bonded to A, the atoms directly bonded to A are different elements, nitrogen and oxygen, and it is possible to consider the possibility that the carbamate compound is electronically unbalanced and has a structure with active electron motion due to the uneven distribution of the electron cloud. Furthermore, both of these functional groups are groups that have structures in which two or more heteroatoms are contained, and furthermore, these heteroatoms are bonded via one carbon atom. From this viewpoint as well, the structure can be considered as one in which uneven distribution of electron cloud can occur. That is, the structure is considered to be active in electron motion in a double or triple sense. In addition, the molecular structure also allows for an asymmetric structure, which is expected to enable more various active species to be formed. As described above, electronic uneven distribution may become more significant in a case where the structure of the formula (1') is an aromatic structure.

On the other hand, in a case where the structure of the formula (1') is an alicyclic structure, it is expected that various active species attributed to confrontations are formed.

The ability to form such a variety of active species is expected to produce a polymer with a broad molecular weight distribution. Also, the electronic uneven distribution is expected to activate appropriate electron donation to the titanium compound component, which will be described later. This is probably one of the reasons for the higher polymerization activity. In addition, such high activity also enables active species to be formed whose molecular weight can be increased, and therefore, as will be described later, the use of the solid titanium catalyst component of the present invention can be considered to easily provide an olefin polymer with a molecular weight distribution that spreads toward the high molecular weight side.

Meanwhile, if too many diverse active species are formed, it may be easier to take active species with low activity and stereospecificity. For this reason, it may be preferable for the A substituent in the formula (1') to have a rigid structure to some extent.

Examples of such carbamate compounds include the following structures. Some structural formulae of the following exemplary compounds have stereoisomers, and even isomeric structures are clearly depicted for some exemplary compounds, but there may be isomeric structures that are not exemplified.

[Chem. 6]

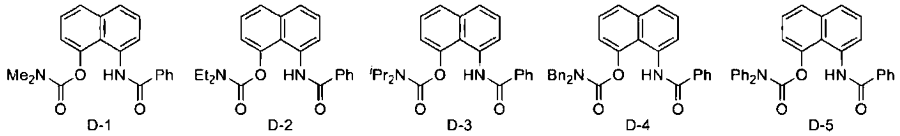

D-1 D-2 D-3 D-4 D-5

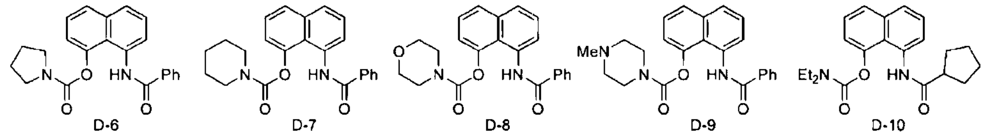

D-6 D-7 D-8 D-9 D-10

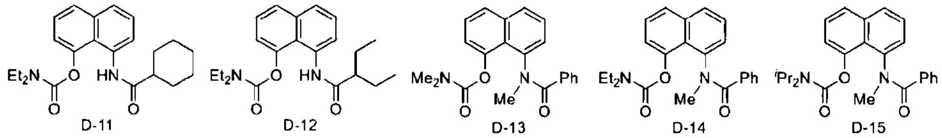

D-11 D-12 D-13 D-14 D-15

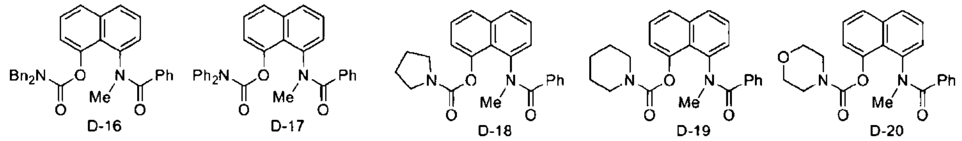

D-16 D-17 D-18 D-19 D-20

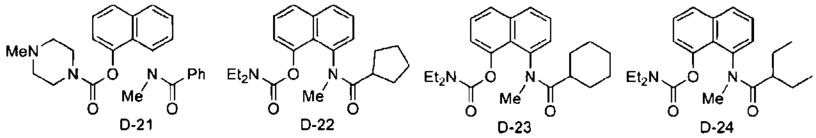

D-21 D-22 D-23 D-24

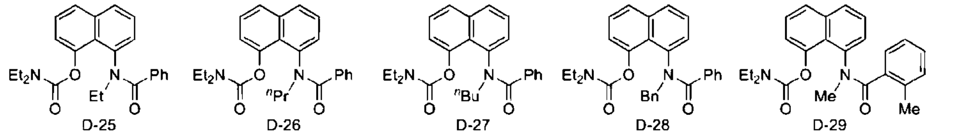

D-25 D-26 D-27 D-28 D-29

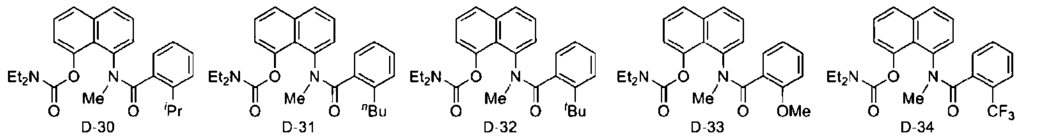

D-30 D-31 D-32 D-33 D-34

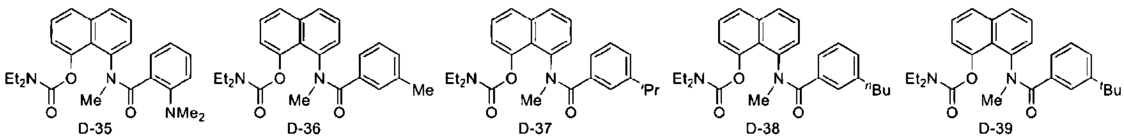

D-35 D-36 D-37 D-38 D-39

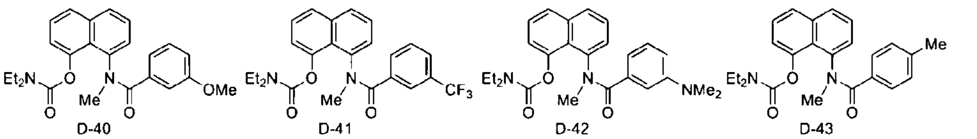

D-40 D-41 D-42 D-43

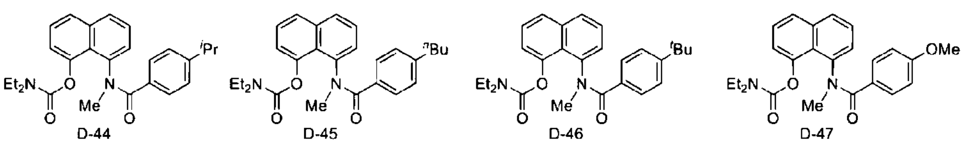

D-44 D-45 D-46 D-47

[Chem. 7]

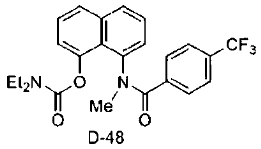
D-48
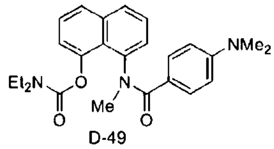
D-49
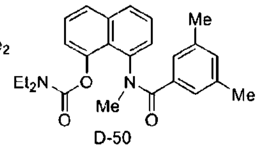
D-50
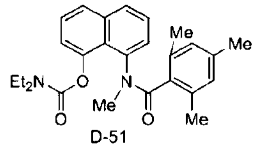
D-51

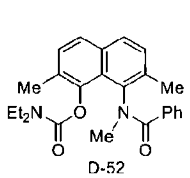
D-52
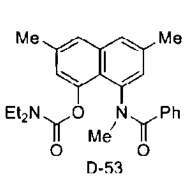
D-53
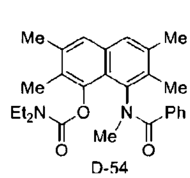
D-54
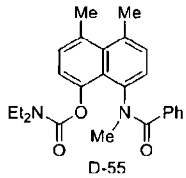
D-55
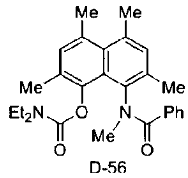
D-56

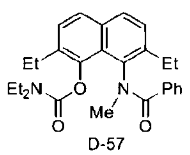
D-57
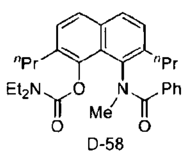
D-58
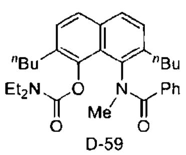
D-59
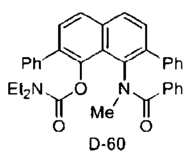
D-60
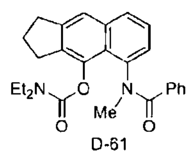
D-61

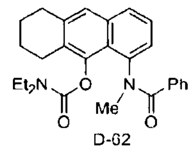
D-62
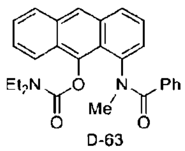
D-63
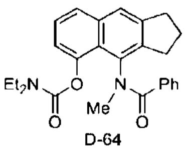
D-64
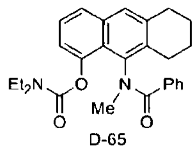
D-65
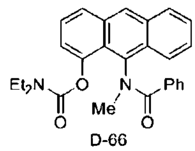
D-66

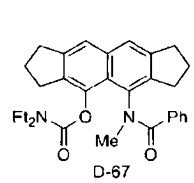
D-67
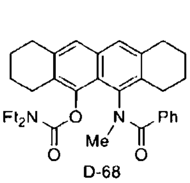
D-68
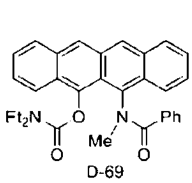
D-69
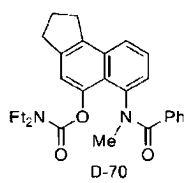
D-70
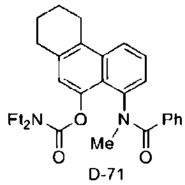
D-71

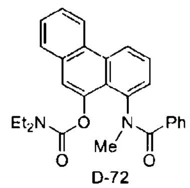
D-72
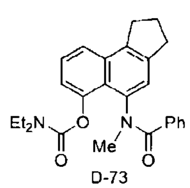
D-73
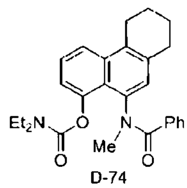
D-74
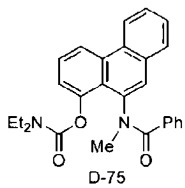
D-75
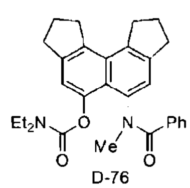
D-76

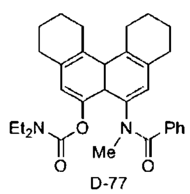
D-77
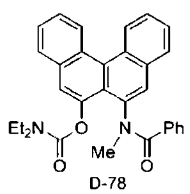
D-78
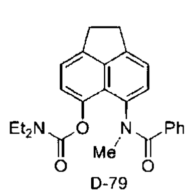
D-79
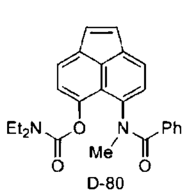
D-80
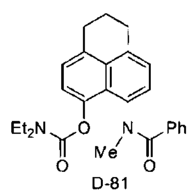
D-81

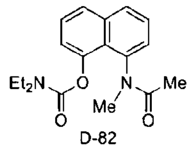
D-82
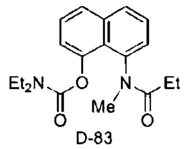
D-83
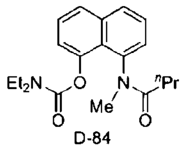
D-84
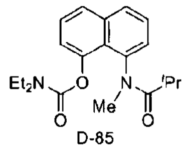
D-85
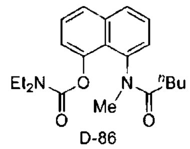
D-86

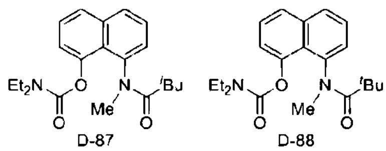
D-87
D-88

[Chem. 8]

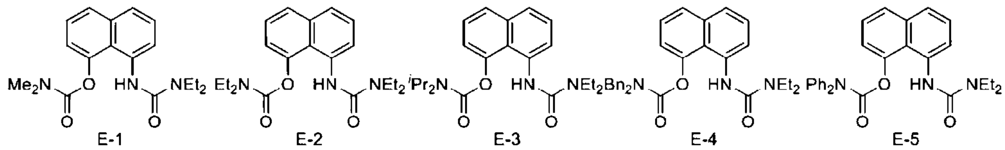

E-1 E-2 E-3 E-4 E-5

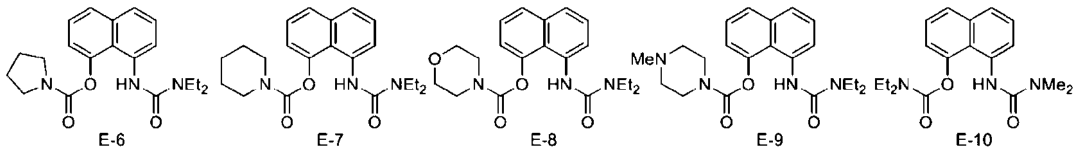

E-6 E-7 E-8 E-9 E-10

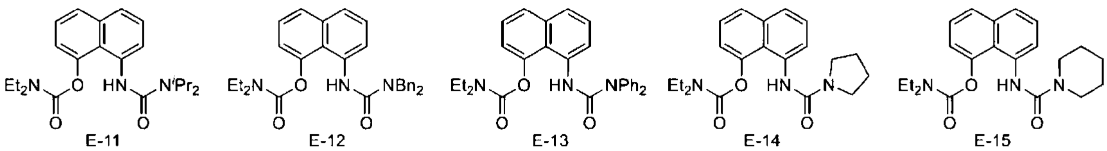

E-11 E-12 E-13 E-14 E-15

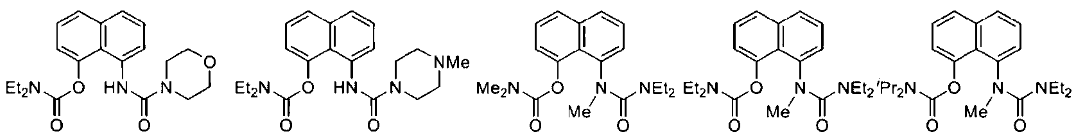

E-16 E-17 E-18 E-19 E-20

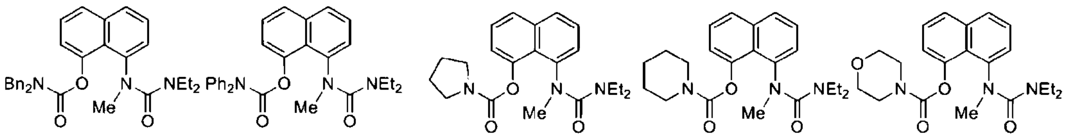

E-21 E-22 E-23 E-24 E-25

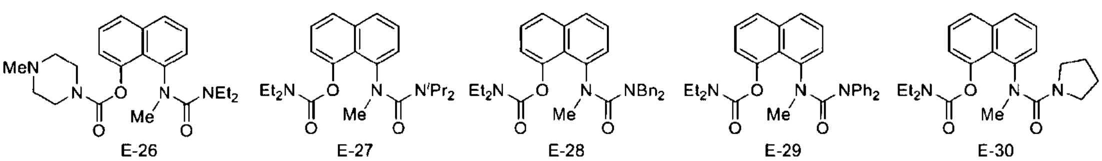

E-26 E-27 E-28 E-29 E-30

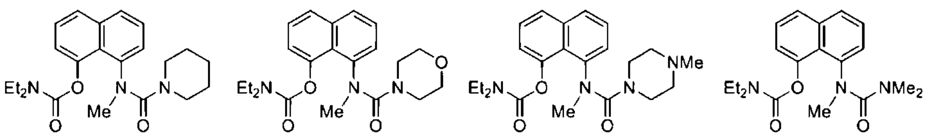

E-31 E-32 E-33 E-34

[Chem. 9]

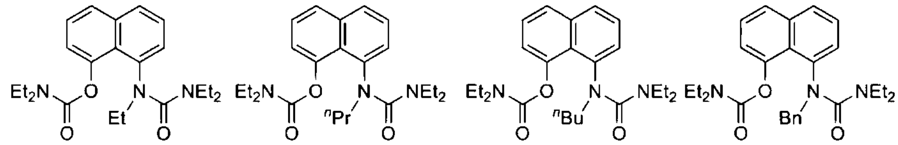

E-35 E-36 E-37 E-38

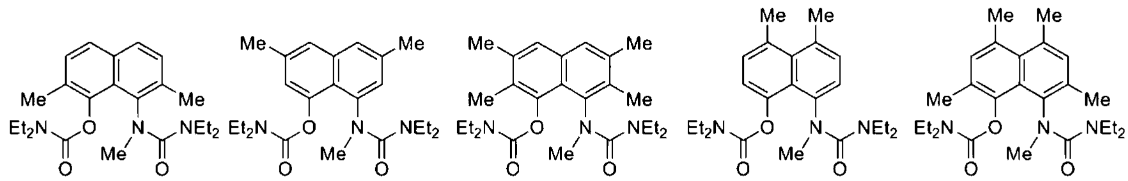

E-39 E-40 E-41 E-42 E-43

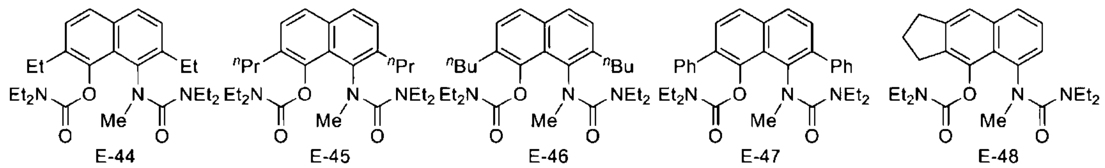

E-44 E-45 E-46 E-47 E-48

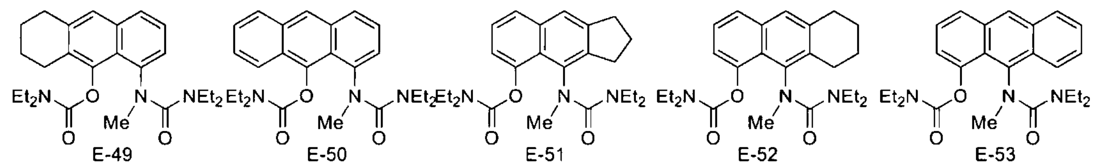

E-49 E-50 E-51 E-52 E-53

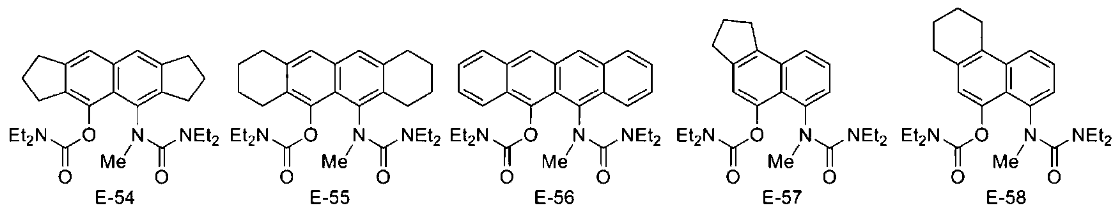

E-54 E-55 E-56 E-57 E-58

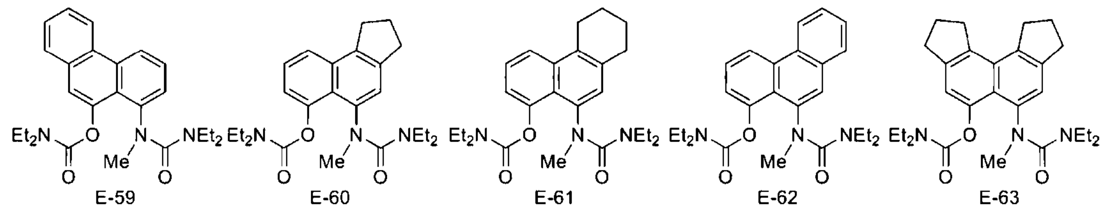

E-59 E-60 E-61 E-62 E-63

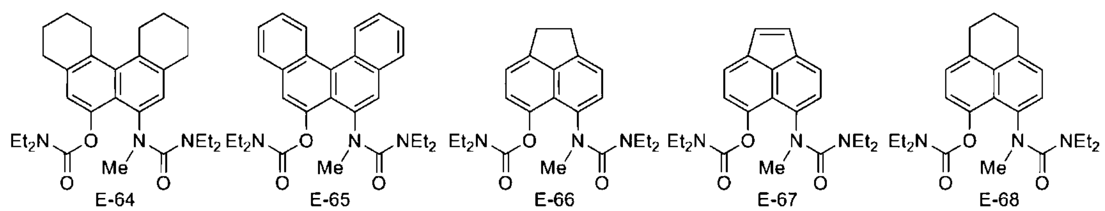

E-64 E-65 E-66 E-67 E-68

[Chem. 10]

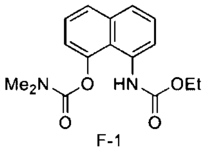
F-1
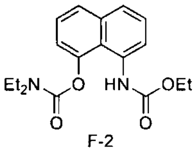
F-2
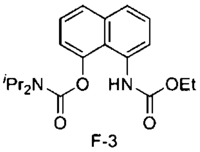
F-3
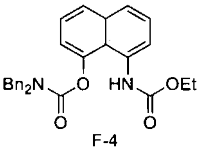
F-4
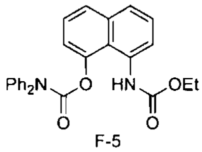
F-5
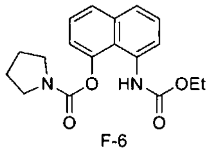
F-6
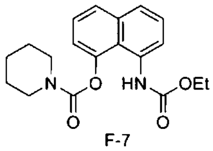
F-7
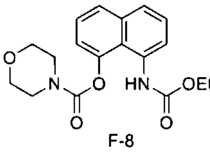
F-8
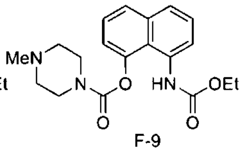
F-9
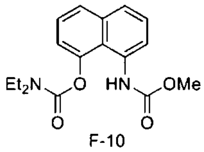
F-10
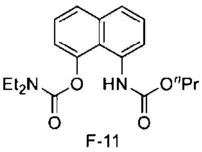
F-11
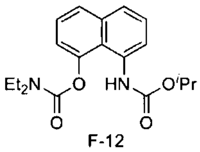
F-12
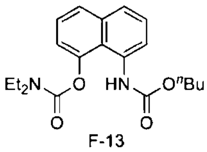
F-13
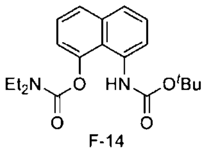
F-14
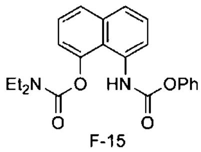
F-15
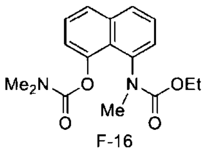
F-16
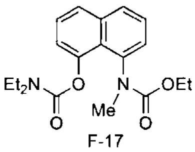
F-17
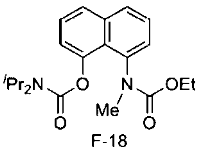
F-18
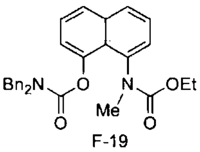
F-19
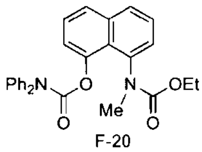
F-20
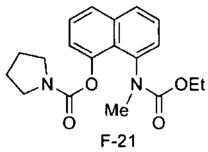
F-21
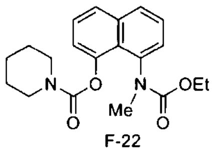
F-22
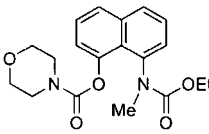
F-23
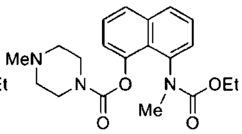
F-24
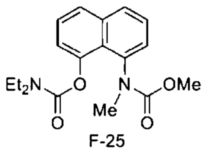
F-25
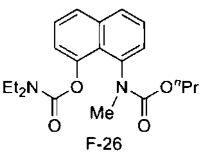
F-26
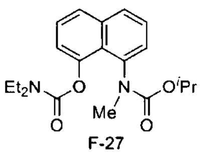
F-27
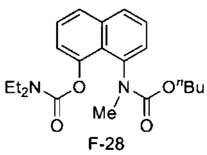
F-28
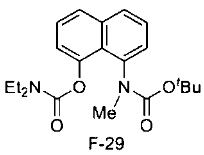
F-29
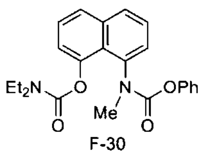
F-30
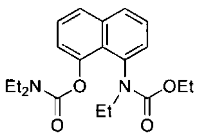
F-31
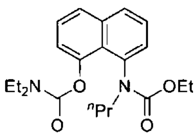
F-32
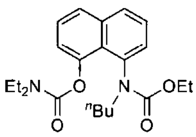
F-33
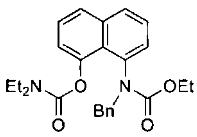
F-34

[Chem. 11]

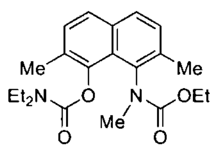
F-35
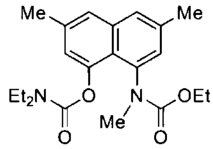
F-36
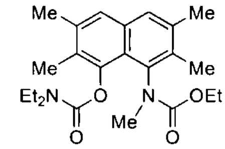
F-37
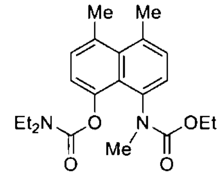
F-38
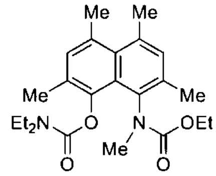
F-39

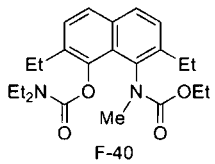
F-40
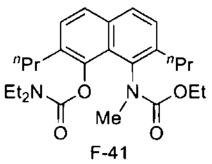
F-41
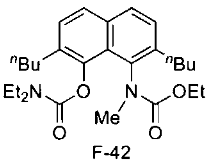
F-42
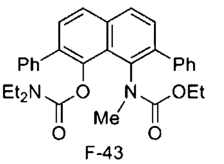
F-43
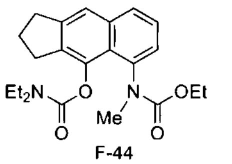
F-44

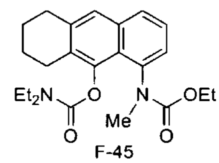
F-45
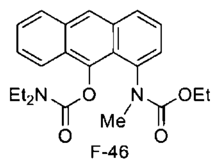
F-46
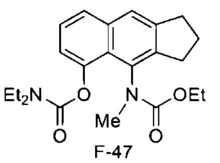
F-47
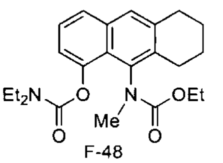
F-48
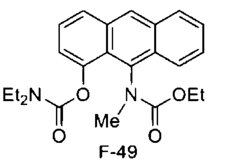
F-49

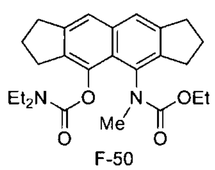
F-50
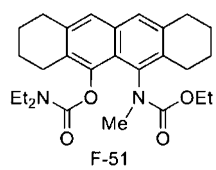
F-51
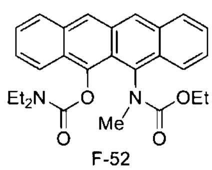
F-52
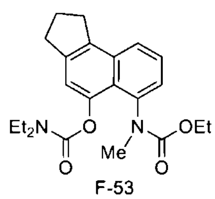
F-53
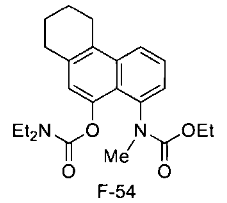
F-54

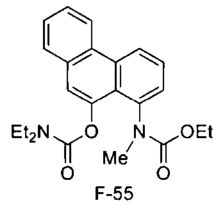
F-55
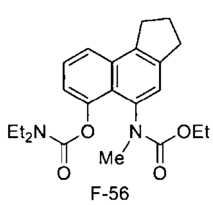
F-56
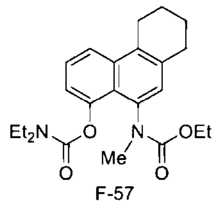
F-57
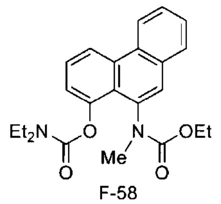
F-58
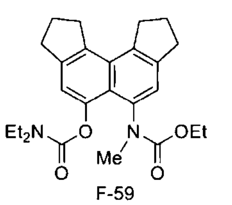
F-59

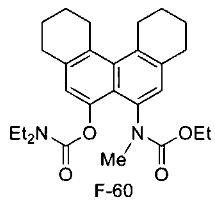
F-60
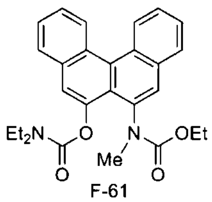
F-61
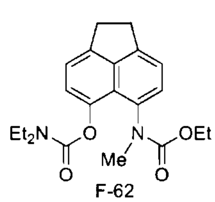
F-62
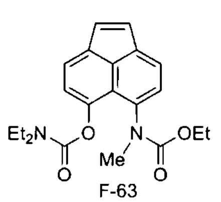
F-63
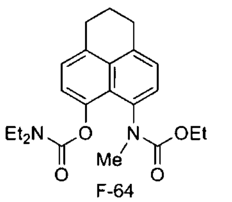
F-64

[Chem. 12]

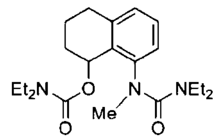
Q-161
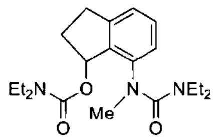
Q-162
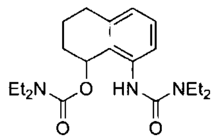
Q-163
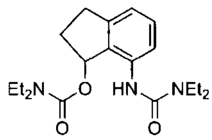
Q-164
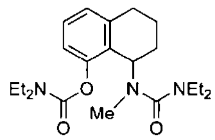
Q-165

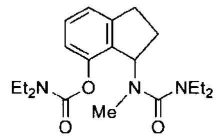
Q-166
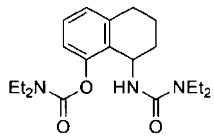
Q-167
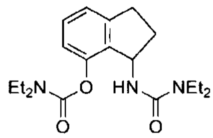
Q-168
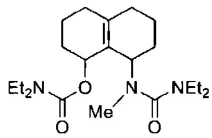
Q-169
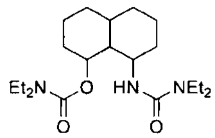
Q-170

[Chem. 13]

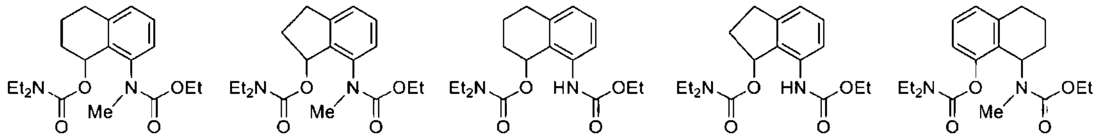

R-161 R-162 R-163 R-164 R-165

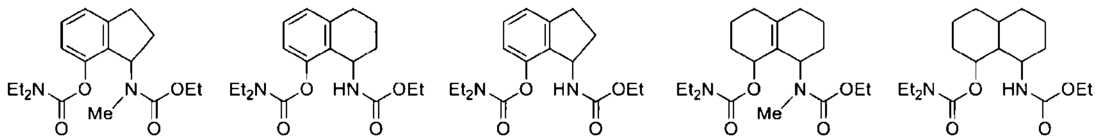

R-166 R-167 R-168 R-169 R-170

[Chem. 14]

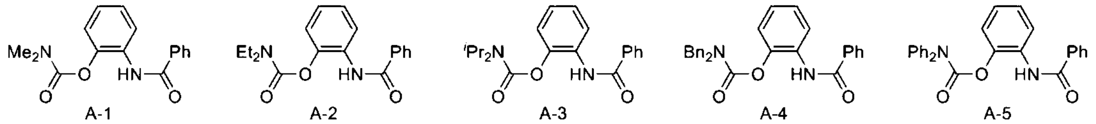

A-1 A-2 A-3 A-4 A-5

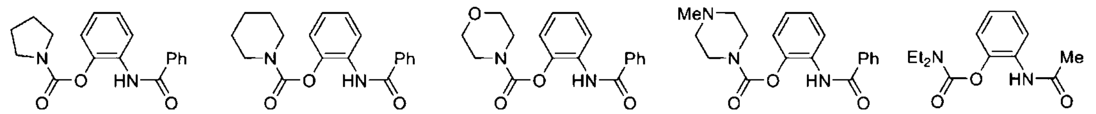

A-6 A-7 A-8 A-9 A-10

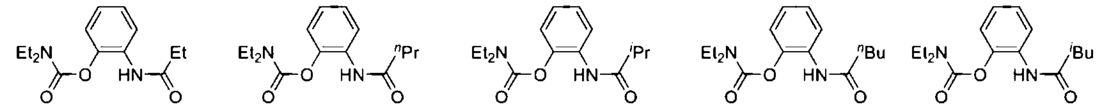

A-11 A-12 A-13 A-14 A-15

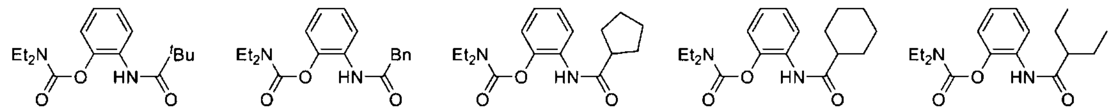

A-16 A-17 A-18 A-19 A-20

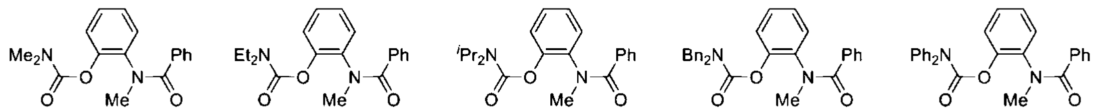

A-21 A-22 A-23 A-24 A-25

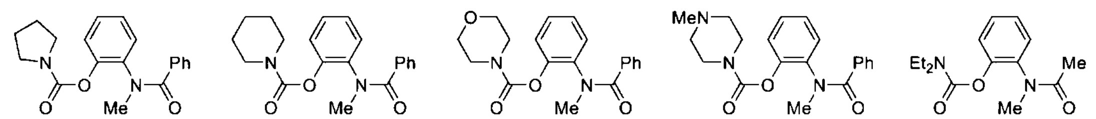

A-26 A-27 A-28 A-29 A-30

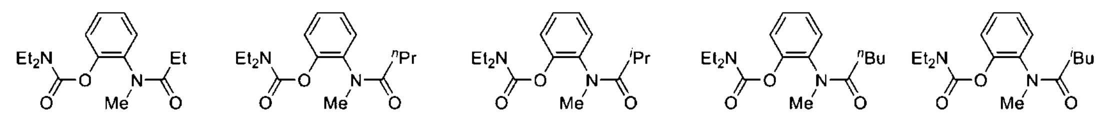

A-31 A-32 A-33 A-34 A-35

[Chem. 15]

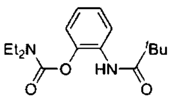
A-36

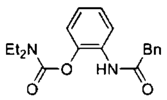
A-37

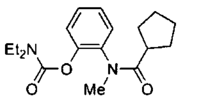
A-38

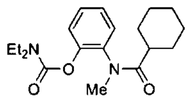
A-39

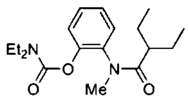
A-40

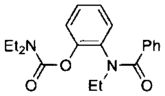
A-41

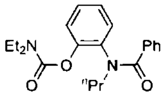
A-42

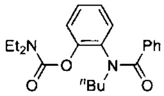
A-43

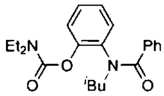
A-44

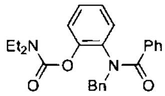
A-45

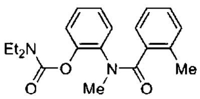
A-46

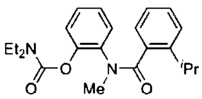
A-47

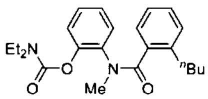
A-48

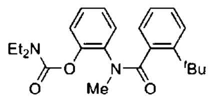
A-49

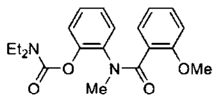
A-50

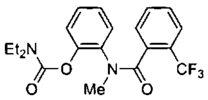
A-51

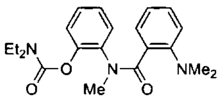
A-52

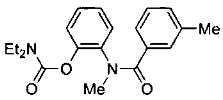
A-53

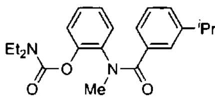
A-54

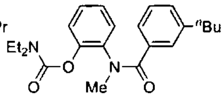
A-55

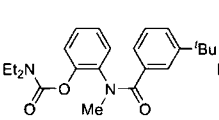
A-56

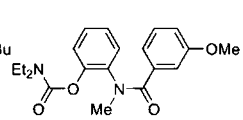
A-57

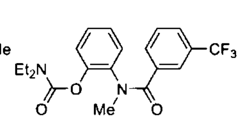
A-58

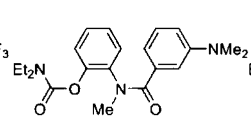
A-59

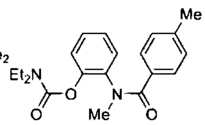
A-60

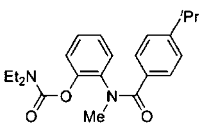
A-61

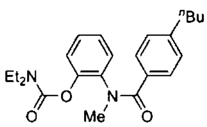
A-62

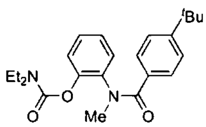
A-63

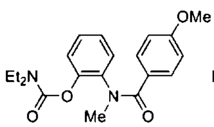
A-64

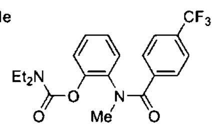
A-65

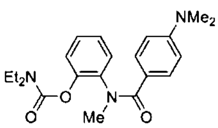
A-66

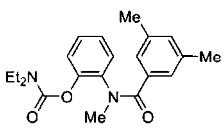
A-67

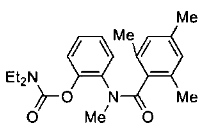
A-68

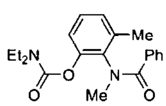
A-69

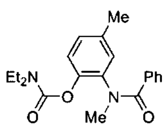
A-70

[Chem. 16]

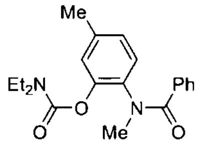
A-71

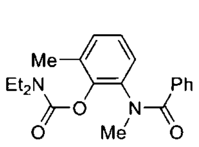
A-72

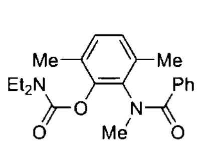
A-73

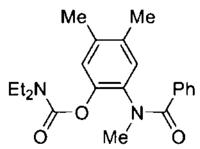
A-74

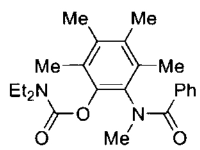
A-75

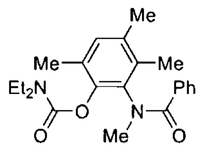
A-76

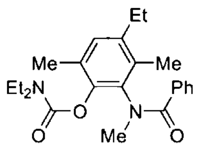
A-77

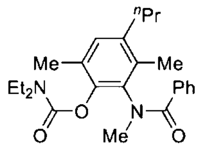
A-78

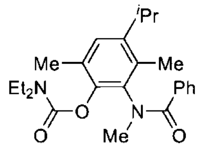
A-79

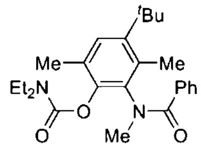
A-80

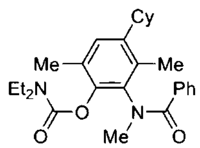
A-81

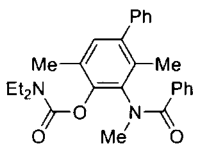
A-82

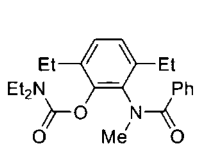
A-83

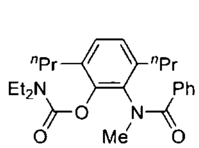
A-84

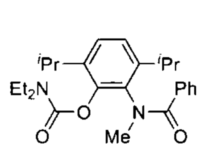
A-85

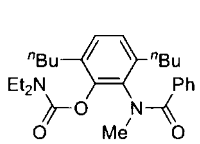
A-86

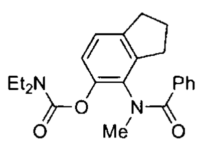
A-87

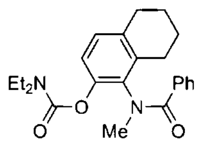
A-88

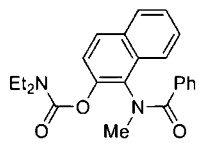
A-89

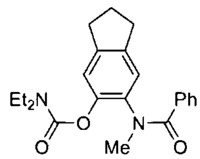
A-90

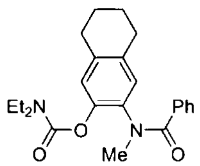
A-91

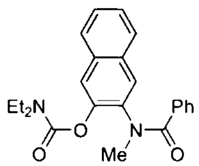
A-92

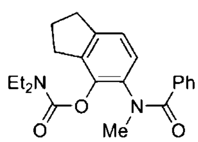
A-93

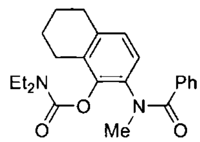
A-94

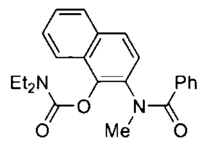
A-95

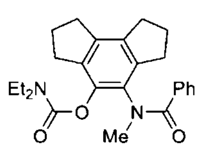
A-96

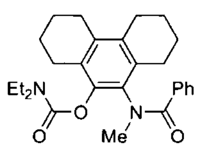
A-97

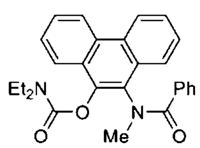
A-98

[Chem. 17]

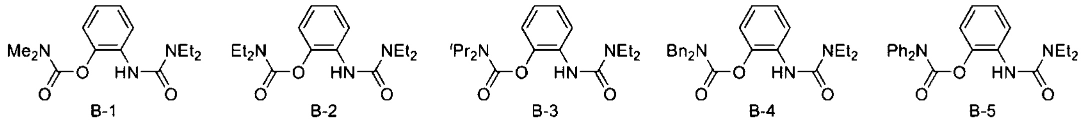

B-1 B-2 B-3 B-4 B-5

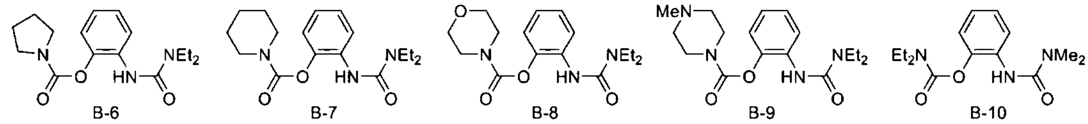

B-6 B-7 B-8 B-9 B-10

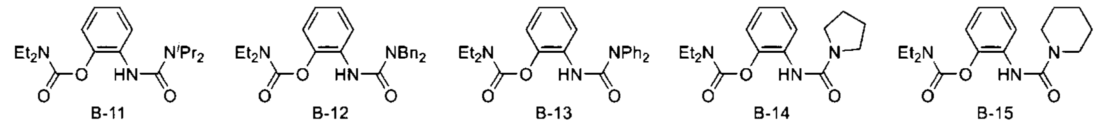

B-11 B-12 B-13 B-14 B-15

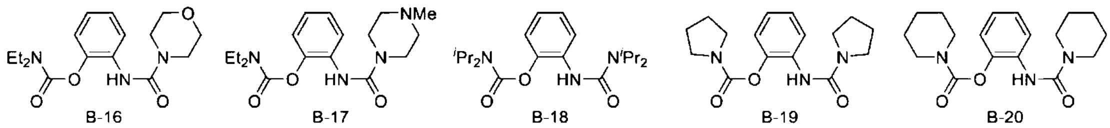

B-16 B-17 B-18 B-19 B-20

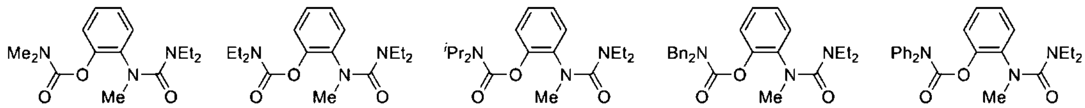

B-21 B-22 B-23 B-24 B-25

[Chem. 18]

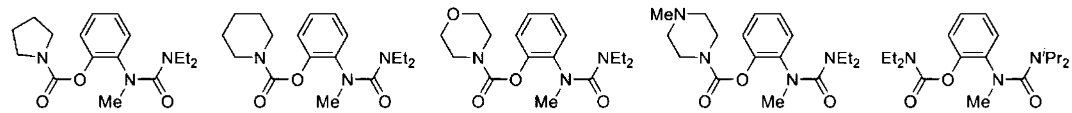

B-26 B-27 B-28 B-29 B-30

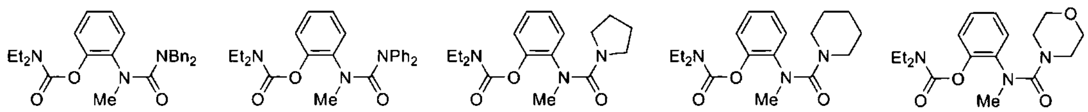

B-31 B-32 B-33 B-34 B-35

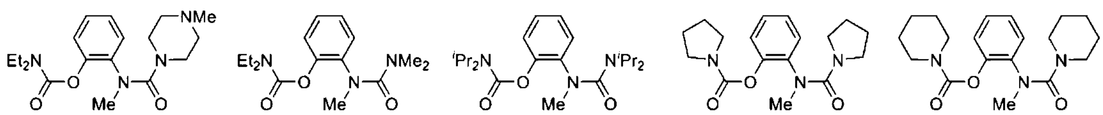

B-36 B-37 B-38 B-39 B-40

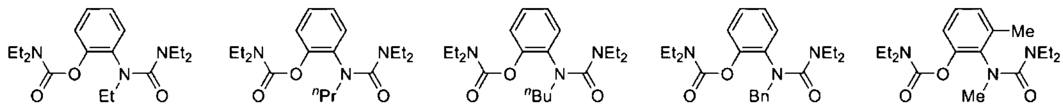

B-41 B-42 B-43 B-44 B-45

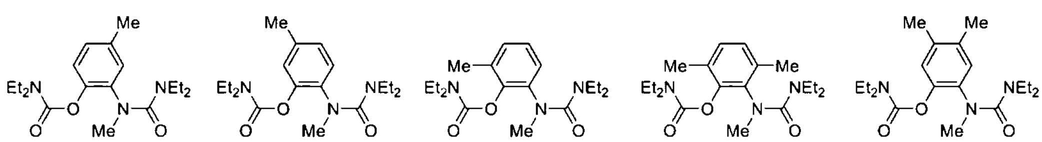

B-46 B-47 B-48 B-49 B-50

[0068]

[Chem. 19]

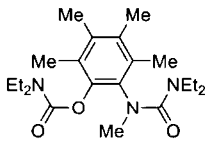
B-51

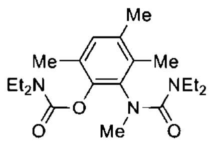
B-52

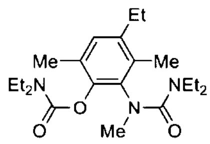
B-53

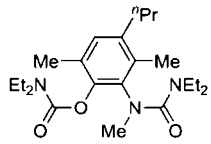
B-54

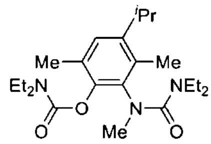
B-55

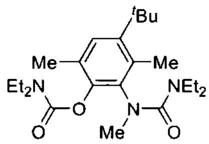
B-56

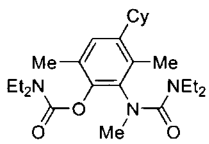
B-57

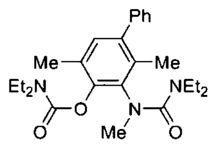
B-58

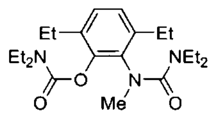
B-59

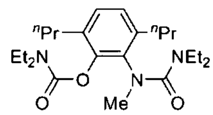
B-60

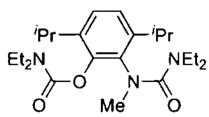
B-61

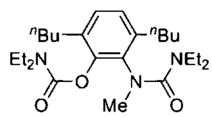
B-62

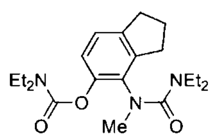
B-63

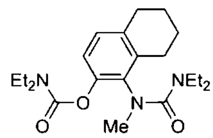
B-64

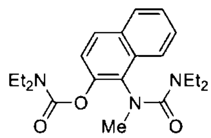
B-65

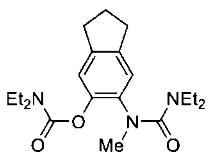
B-66

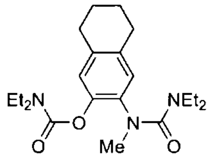
B-67

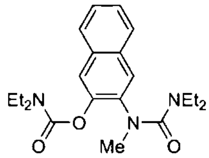
B-68

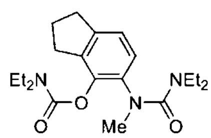
B-69

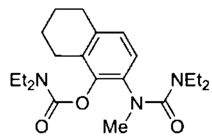
B-70

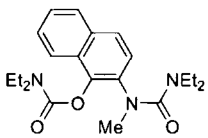
B-71

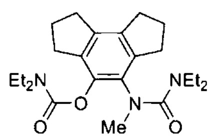
B-72

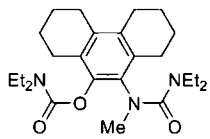
B-73

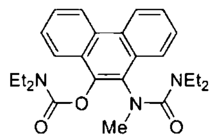
B-74

[Chem. 20]

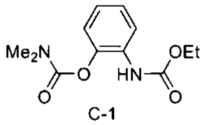
C-1

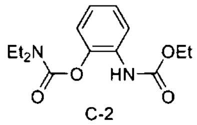
C-2

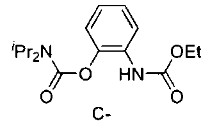
C-
3

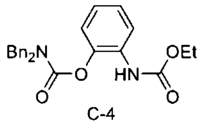
C-4

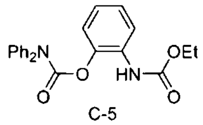
C-5

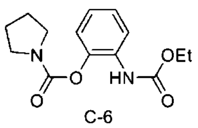
C-6

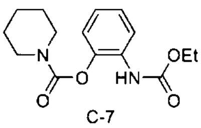
C-7

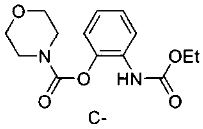
C-
8

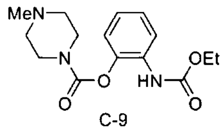
C-9

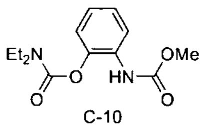
C-10

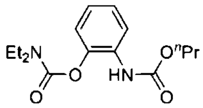
C-11

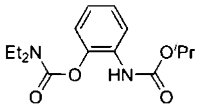
C-12

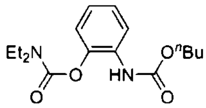
C-13

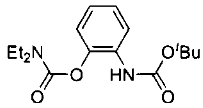
C-14

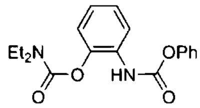
C-15

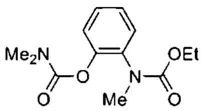
C-16

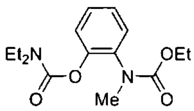
C-17

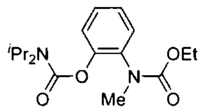
C-18

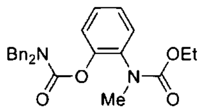
C-19

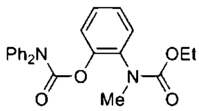
C-20

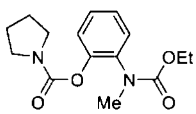
C-21

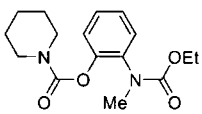
C-22

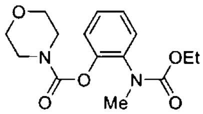
C-23

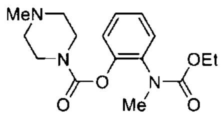
C-24

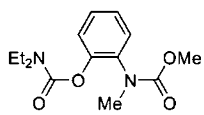
C-25

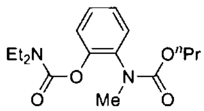
C-26

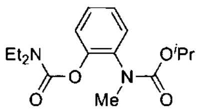
C-27

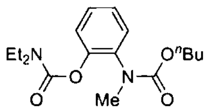
C-28

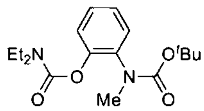
C-29

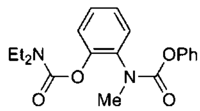
C-30

[Chem. 21]

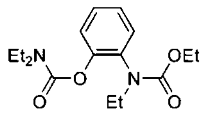
C-31

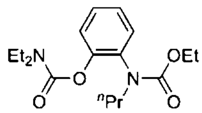
C-32

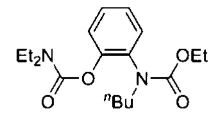
C-33

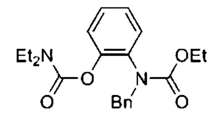
C-34

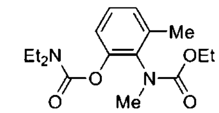
C-35

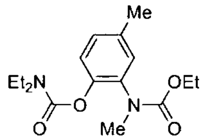
C-36

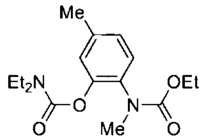
C-37

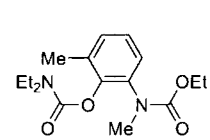
C-38

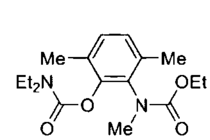
C-39

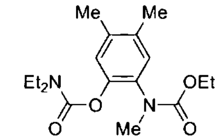
C-40

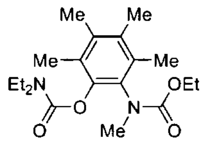
C-41

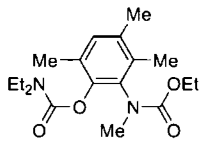
C-42

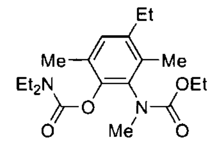
C-43

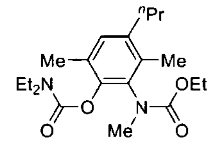
C-44

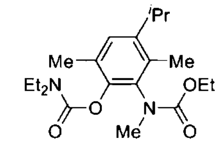
C-45

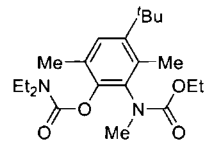
C-46

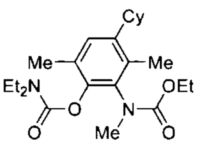
C-47

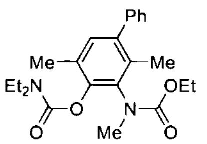
C-48

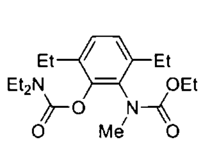
C-49

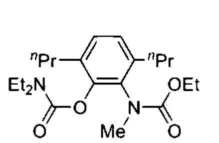
C-50

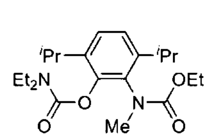
C-51

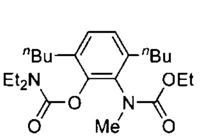
C-52

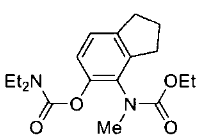
C-53

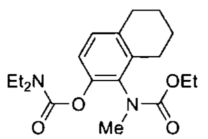
C-54

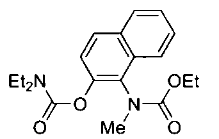
C-55

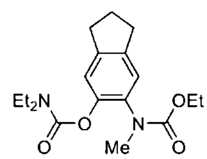
C-56

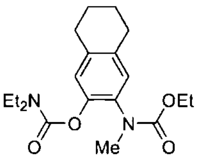
C-57

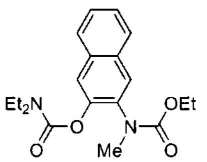
C-58

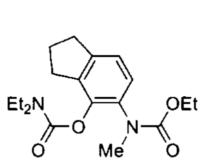
C-59

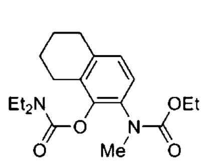
C-60

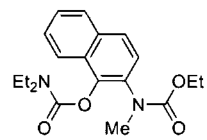
C-61

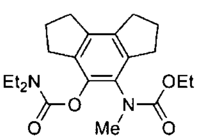
C-62

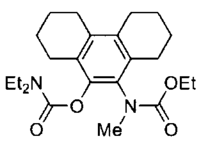
C-63

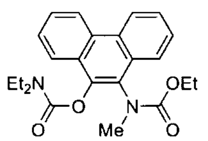
C-64

In the structural formulae, the methyl group is indicated by "Me," the ethyl group is indicated by "Et," the propyl group is indicated by "Pr," the butyl group is indicated by "Bu," the phenyl group is indicated by "Ph," the benzyl group is indicated by "Bn," the cyclohexyl group is indicated by "Cy," and the trifluoromethyl group is indicated by "$CF_3$." "n" represents "normal," "i" represents "iso," and "t" represents "tertiary."

A carbon atom is present at the intersection or the end portion of the line in the structure. Such a method for expressing compound structures is a method well known to those skilled in the art.

<Method for producing carbamate compound>

A method for producing a carbamate compound of the present invention is not particularly limited, and it is possible to use, for example, "synthesis examples" in Examples described below. The carbamate compound may also be produced using a known reaction. The carbamate compound may also be synthesized by synthesizing each moiety by a known synthesis method and combining them by a known method. More specifically, it is possible to exemplify a method in which the carbamate compound is synthesized by synthesis of an amide alcohol as described below. In the following formulae, R' refers to a substituent that bonds by an adjacent atom and a carbon atom.

(Method for synthesizing N-amide moiety)

In the following reaction formula (2), an amide compound can be synthesized by, for example, a reaction between an amino alcohol compound and 1 equivalent of an acid chloride in the presence of a base. The amino alcohol hydrochloride can be used in place of the amino alcohol. Examples of the base used can include, but not limited to, sodium hydroxide, potassium hydroxide, pyridine, N,N-dimethyl-4-aminopyridine, and triethylamine.

[Chem. 22]

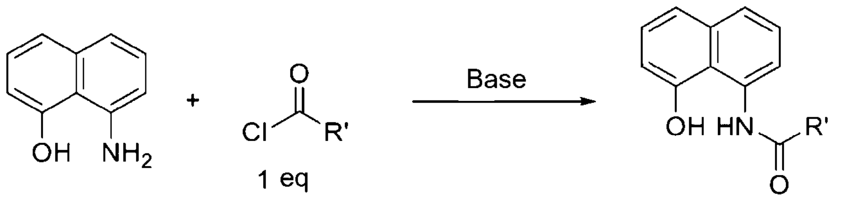


(2)

Alternatively, the amide compound that corresponds to the reaction formula (2) can be synthesized by synthesizing an N-amide/O-ester compound by a reaction between an amino alcohol compound and 2 equivalents or more of an acid chloride in the presence of a base and subsequently reacting the N-amide/O-ester compound with the base as shown in the following reaction formula (3). The amino alcohol used in synthesis of the N-amide/O-ester compound may be a corresponding hydrochloride, and examples of the base used can include, but not limited to, sodium hydroxide, potassium hydroxide, pyridine, N,N-dimethyl-4-aminopyridine, and triethylamine. Examples of a method for synthesizing the N-amide/O-ester compound include the method including reacting an amino alcohol compound with carboxylic acid in the presence of an acid catalyst or the method using a condensing reagent, such as N,N'-dicyclohexylcarbodiimide (DCC) (see the following reaction formula (4)). Examples of the base that can be used in synthesis of the amide compound include, but not limited to, inorganic bases, such as sodium hydroxide and potassium hydroxide, and organic bases, such as piperidine and pyrrolidine.

[Chem. 23]

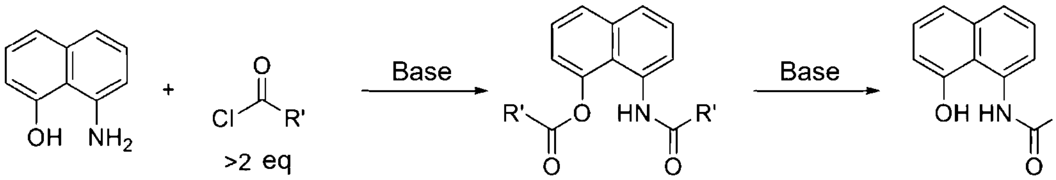


(3)

[Chem. 24]

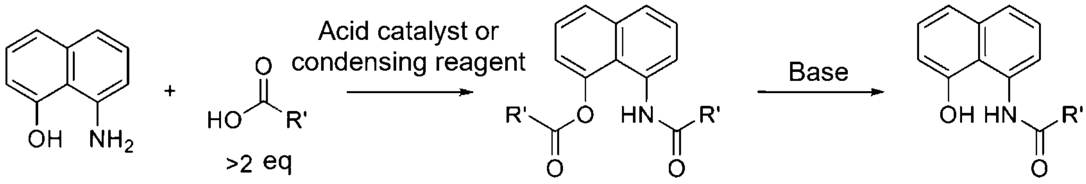


(4)

(Method for synthesizing N-carbamate moiety)

A N-carbamate compound shown in the following reaction formula (5) can be synthesized by, for example, a reaction between an amino alcohol compound and 1 equivalent of a chloroformate in the presence of a base. The amino alcohol used may be a corresponding hydrochloride. Examples of the base can include, but not limited to, pyridine, N,N-dimethyl-4-aminopyridine, and triethylamine.

[Chem. 25]

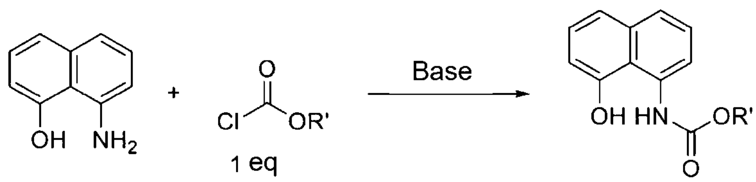


(5)

Alternatively, the N-carbamate compound that corresponds to the reaction formula (5) can be synthesized by synthesizing a N-carbamate/O-carbonate compound by a reaction between an amino alcohol compound and 2 equivalents or more of a chloroformate in the presence of a base and subsequently reacting the N-carbamate/O-carbonate compound with the base as shown in the following reaction formula (6). The amino alcohol used in synthesis of the N-carbamate/O-carbonate compound may be a corresponding hydrochloride, and examples of the base used can include, but not limited to, pyridine, N,N-dimethyl-4-aminopyridine, and triethylamine. Examples of the base that can be used in synthesis of the N-carbamate compound include, but not limited to, inorganic bases, such as sodium hydroxide and potassium hydroxide, and organic bases, such as piperidine and pyrrolidine.

[Chem. 26]

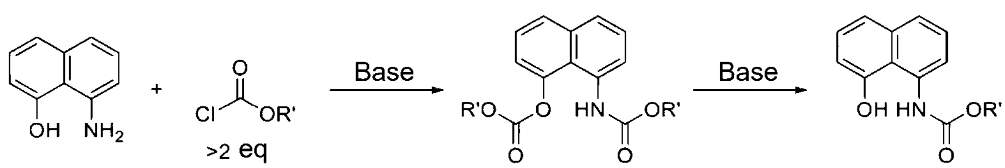


(6)

(Method for synthesizing diamino carbonyl moiety)

A diaminocarbonyl compound shown in the following formula (7) can be synthesized by, for example, a reaction between an amino alcohol compound and 1 equivalent of carbamoyl chloride in the presence of a base. The amino alcohol used may be a corresponding hydrochloride. Examples of the base can include, but not limited to, sodium hydroxide, potassium hydroxide, pyridine, N,N-dimethyl-4-aminopyridine, and triethylamine.

[Chem. 27]

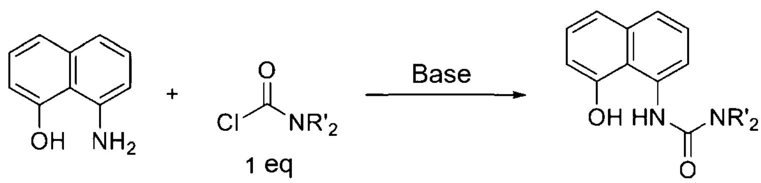


(7)

Alternatively, a diaminocarbonyl compound shown in the following reaction formula (7) can also be synthesized by a reaction between an amino alcohol compound and 1 equivalent of an imidazolium salt in the presence of a base as shown in the following reaction formula (8). The amino alcohol used may be a corresponding hydrochloride. Examples of the base can include, but not limited to, pyridine, N,N-dimethyl-4-aminopyridine, triethylamine, and n-butyllithium.

[Chem. 28]

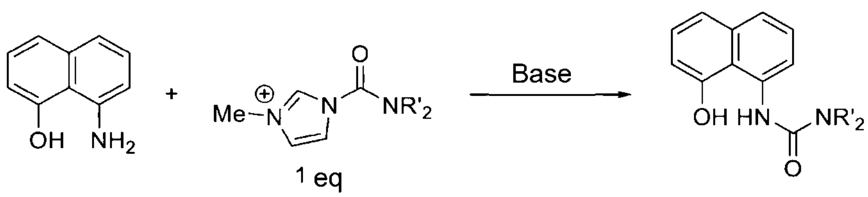


(8)

(Method for synthesizing O-carbamate moiety)

A compound shown in the following reaction formula (9) can be synthesized by, for example, a reaction between one of the above-described phenols and a chloroformate in the presence of a base. Examples of the base can include, but not limited to, pyridine, N,N-dimethyl-4-aminopyridine, and triethylamine.

[Chem. 29]

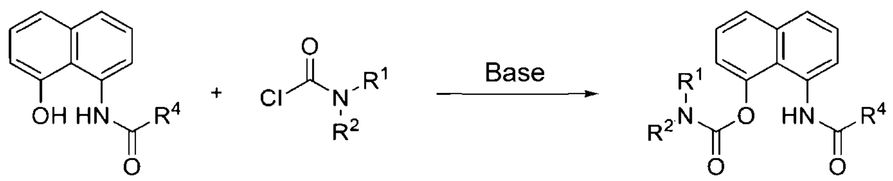


(9)

(Method for synthesizing N-alkyl moiety)

A compound shown in the following reaction formula (10) can be synthesized by, for example, a reaction between the above-described compound and a base and a subsequent reaction with an alkyl halide. Examples of the base can include, but not limited to, organolithium reagents and sodium hydride.

[Chem. 30]

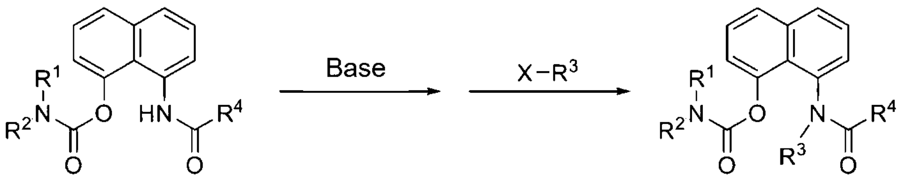


(10)

These compounds can be used in various applications described below. At that time, one of these compounds may be used singly, or two or more thereof may be used in combination. These carbamate compounds can also be jointly used with other compounds as long as the object of the present invention is not impaired. For example, in the case of being used as an internal donor of a solid titanium catalyst component, the carbamate compound may be used in combination with one of the alcohols described below or a known electron donor, such as an ester or an ether.

When used as a solid titanium catalyst component, the carbamate compound may be formed in the process of preparing the solid titanium catalyst component.

As the applications of the carbamate compound of the present invention, not only an internal donor component of a solid titanium catalyst component that is used as the olefin polymerization catalyst but also drug applications, such as not only pesticides but also intermediates or raw materials of medicines and pesticides, can be expected. Since the carbamate compound can be expected to be a structure having a unique polarity, the carbamate compound can also be expected to be suitable as a raw material of a column filler of, for example, an optical isomer separation (a chiral separation column) column. The carbamate compound can also be considered as, possibly, a raw material of a polycondensation product, such as nylon.

Examples

(Method for analyzing compounds)

A $^1$H-NMR spectrum (400 MHz, manufactured by JEOL Ltd., JNM-ECZ400S/L1 type measuring instrument) was measured, the peaks were assigned by a routine method to determine a structure.

[Example 1]

<Synthesis of compound 1>

A compound 1 shown below was synthesized by a method described below according to the following reaction

formula.

[Chem. 31]

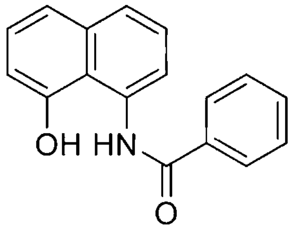

(Compound 1)

[Chem. 32]

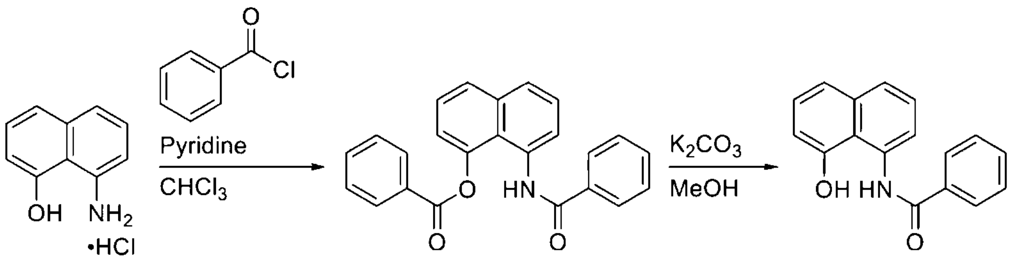


Intermediate 1′

Compound 1

2.93 g of 8-amino-1-naphthol hydrochloride (15.0 mmol, 1 eq) and 60 mL of chloroform (dehydrated) were added into an oven-dried 300 mL three-necked flask containing a magnetic stirring bar in a nitrogen atmosphere. After that, 6.04 mL of pyridine (dehydrated) (75.0 mmol, 5 eq) was slowly added dropwise thereto at room temperature. After the reaction solution was cooled in an ice bath, 3.87 mL of benzoyl chloride (33.0 mmol, 2.2 eq) was slowly added dropwise thereto. After the end of the dropwise addition, the reaction solution was heated to room temperature and stirred for five hours. After the end of a reaction, the reaction solution was cooled again in an ice bath, 15 mL of methanol was added thereto, and the reaction solution was stirred at room temperature for 30 minutes. Water, dichloromethane, and methanol were added to the reaction solution, and extraction was then performed three times with the dichloromethane. A combined organic layer was dried over sodium sulfate and concentrated with a rotary evaporator. 8.81 g of the obtained crude product was purified by silica gel column chromatography (eluent, hexane: dichloromethane = gradient from 50:50 to 0:100) to obtain 3.60 g of the intermediate 1'. 3.60 g of the intermediate 1', 70 mL of methanol, and 1.76 g of potassium carbonate (12.7 mmol) were added into a 500 mL round-bottom flask, and the solution were stirred at 40°C for two hours. After the end of a reaction, water and dichloromethane were added thereto, and extraction was performed three times with the dichloromethane. Furthermore, the aqueous layer was acidified by adding ammonium chloride thereto, and extraction was then performed again three times with the dichloromethane. A combined organic layer was dried over sodium sulfate and then concentrated with the rotary evaporator. The organic layer was suspended with hexane, filtered, and purified to obtain 2.53 g of the compound 1 (9.61 mmol, yield 64%).

<Synthesis of compound 2>

A compound 2 shown below was synthesized by a method described below.

[Chem. 33]

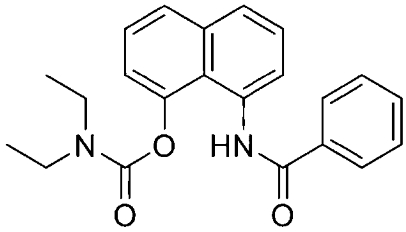

(Compound 2)

27.5 g of the compound 1 (104.4 mmol, 1 eq) and 349 mL of pyridine were added into an oven dried 1 L three-necked flask containing magnetic stirring bar in a nitrogen atmosphere and stirred at room temperature. 33.1 mL of diethylcarbamoyl chloride (261.1 mmol, 2.5 eq) was slowly added dropwise thereto. After the completion of the dropwise addition, the reaction solution was refluxed for one hour. After the end of a reaction, the reaction solution was cooled to room temperature. Next, the reaction solution was added to 800 mL of ice-cooled distilled water. Extraction was performed twice with diethyl ether, and an combined organic layer was washed once with each of 1N hydrochloric acid and a saturated sodium bicarbonate aqueous solution and dried over anhydrous magnesium sulfate. The organic layer was concentrated with a rotary evaporator to obtain 23.8 g of a crude product. As a result of purifying the obtained crude product by silica gel column chromatography (eluent, hexane: ethyl acetate = gradient from 4:1 to 2:1) to obtain 15.1 g of the compound 2 (41.7 mmol, yield 40%).

<Preparation of solid titanium catalyst component [α1]>

After a 1-liter glass vessel was sufficiently substituted with nitrogen, 85.8 g of anhydrous magnesium chloride, 321 g of decane, and 352 g of 2-ethylhexyl alcohol were put thereinto and heated and reacted at 130°C for three hours to produce a homogeneous solution. 241 g of this solution and 6.43 g of ethyl benzoate were added to a glass container and stirred and mixed at 50°C for one hour.

After the homogeneous solution thus obtained was cooled to room temperature, 38.3 ml of this homogeneous solution was loaded in its entirety by dropwise addition into 100 ml of titanium tetrachloride retained at -20°C over 45 minutes while being stirred. After the end of the loading, the temperature of this mixed liquid was raised to 80°C over 3.8 hours, and when the temperature reached 80°C, 1.83 g of the compound 2 was added to the mixed liquid. The temperature was raised again to 120°C over 40 minutes, and a mixture was retained at the same temperature for 35 minutes while being stirred. After the end of a reaction, a solid portion was recovered by hot filtration, this solid portion was suspended again in 100 ml of titanium tetrachloride, and a heating reaction was performed again at 120°C for 35 minutes while the solid portion was stirred. After the end of the reaction, the solid portion was recovered again by hot filtration and sufficiently washed with 100°C decane and room temperature decane until no free titanium compound was detected in the washing liquid. A solid titanium catalyst component [α1] prepared by the above-described operation was preserved as a decane slurry, and a part of this slurry was dried to check the catalyst composition. The composition of the solid titanium catalyst component [α1] thus obtained contained 0.42% by mass of titanium, 1.4% by mass of magnesium, and 0.11% by mass of a 2-ethylhexyl alcohol residue.

<Polymerization>

After the addition of 500 g of propylene and 1 NL of hydrogen at room temperature to a polymerization vessel having an inner capacity of 2 L, a mixture obtained by mixing 7 mL of heptane, 0.5 mmol of triethyl aluminum, 0.1 mmol of cyclohexylmethyldimethoxysilane, and 0.004 mmol of the solid titanium catalyst component [α1] (in terms of titanium atom) at 25°C for 10 minutes was added thereto, and the temperature inside the polymerization vessel was rapidly raised up to 70°C under stirring. After polymerization at 70°C for 1.5 hours, the reaction was stopped with a small amount of ethanol, and propylene was purged. Furthermore, obtained polymer particles were dried under reduced pressure at 80°C overnight. For example, the activity, the bulk specific gravity, MFR, the amount of a decane-insoluble part, Tm, Tmf, and MWD are as described below.

Activity: 34.1 kg-PP/g-catalyst
Bulk specific gravity: 470 kg/$m^3$
MFR: 1.1 g/10 minutes
Decane-insoluble part content: 1.72% by mass
Tm: 163.7°C, 148.2°C
Tc: 117.1°C

Tmf: 172.4°C
ΔH: 91.1 J/g
Mw/Mn: 11.22
Mz/Mw: 5.45

Methods for measuring the physical properties described above are as follows.

(1) Bulk specific gravity:
The bulk specific gravity was measured in accordance with JIS K-6721.
(2) Melt flow rate (MFR):
In accordance with ASTM D1238E, the measurement temperature for a propylene polymer was set to 230°C with a load of 2.16 kg.
(3) Amount of decane-soluble (insoluble) part:
A glass measuring container is charged with about 3 gram of the propylene polymer (measured up to an accuracy of $10^{-4}$ g; the weight indicated by b (gram) in the following formula), 500 ml of decane, and a small amount of a heat-resistant stabilizer soluble in decane, and the propylene polymer was dissolved by being heated to 150°C over two hours while being stirred with a stirrer in a nitrogen atmosphere, allowed to stand at 150°C for two hours, and gradually cooled to 23°C over eight hours. A liquid containing an obtained precipitate of the propylene polymer was filtered under reduced pressure with a 25G-4 standard glass filter manufactured by Tokyo Garasu Kikai Co., Ltd. 100 ml of a filtrate was recovered and dried under reduced pressure to obtain a part of a decane-soluble part, and this weight was measured up to an accuracy of $10^{-4}$ g (this weight was indicated by a (g) in the following formula). After this operation, the amount of the decane-soluble part was determined by the following formula.
Decane-soluble part content = 100 × (500 × a)/(100 × b)

Decane-insoluble part content = 100 - 100 × (500 × a)/(100 × b)

(4) Molecular weight distribution (MWD):

Gel permeation chromatograph: HLC-8321 GPC/HT type manufactured by Tosoh Corporation
Detector: Differential refractometer
Column: Two TSKgel GMH6-HTs and two TSKgel GMH6-HTLs manufactured by Tosoh Corporation were serially connected.
Mobile phase medium: o-Dichlorobenzene
Flow rate: 1.0 ml/minute
Measurement temperature: 140°C
Method of creating calibration curve: A standard polystyrene sample was used.
Sample concentration: 0.1% (w/w)
Amount of sample solution: 0.4 ml

Measurement was performed under the above-described conditions, an obtained chromatogram was analyzed by a known method to calculate the weight average molecular weight (Mw), the number average molecular weight (Mn), the Z average molecular weight (Mz), the Mw/Mn value and the Mz/Mw value, which are indices of the molecular weight distribution (MWD). The measurement time per sample was 60 minutes.
(5) Melting point (Tm) of polymer:
The melting point (Tm), crystallization temperature (Tc), and amount of heat of fusion (ΔH) of the polymer in the present invention were measured with a differential scanning calorimeter (DSC) that was a DSC 8000 device manufactured by PerkinElmer Co., Ltd. 3 to 10 mg of a specimen was sealed in an aluminum pan and heated from room temperature to 200°C at 100°C/minute. This specimen was retained at 200°C for five minutes and then cooled to 30°C at 10°C/minute. A peak temperature that was observed in this cooling test was regarded as the crystallization temperature (Tc), and the amount of heat generated that was specified by the area of the peak was regarded as ΔH. Subsequently, the specimen was retained at 30°C for five minutes and then heated for the second time to 200°C at 10°C/minute. A peak temperature that was observed by this second heating test was regarded as the melting point (Tm).

The final melting point (Tmf) of the polymer in the present invention was measured with a differential scanning calorimeter (DSC) that was the DSC 8000 device manufactured by PerkinElmer Co., Ltd. 3 to 10 mg of a specimen was sealed in an aluminum pan and heated from room temperature to 240°C at 80°C/minute. The specimen was retained at

240°C for one minute and then cooled to 0°C at 80°C/minute. The specimen was retained at 0°C for one minute, then heated to 150°C at 80°C/minute, and retained for five minutes. Finally, the specimen was heated to 180°C at 1.35°C/minute, and the intersection between the tangent of the inflection point on the high-temperature side of a peak that was obtained in this final heating test and the base line was employed as the final melting point (Tmf).

Tmf can be considered as one of the parameters for evaluating, for example, the crystal structure of a component exhibiting extremely high stereoregularity or the ease of crystallization or crystal structure of a polymer in an ultra-high molecular weight region, which is considered to have a tendency of being less likely to crystallize. More specifically, it can be considered that as the value of this Tmf is higher, an ultra-high molecular weight polymer component is more likely to form highly heat-resistant crystals.

[Example 2]

<Synthesis of compound 3>

A compound 3 shown below was synthesized by a method described below.

[Chem. 34]

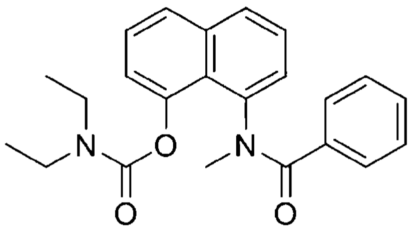

(Compound 3)

15.1 g of the compound 2 (41.7 mmol, 1 eq) and 417 mL of THF (dehydrated) were added into an oven dried 2 L three-necked flask containing a magnetic stirring bar in a nitrogen atmosphere and stirred in an ice bath. 4.55 g of sodium hydride (55%, liquid paraffin dispersion, 104.2 mmol, 2.5 eq) was slowly added thereto and stirred for 30 minutes in an ice bath. Next, 5.19 mL of iodomethane (83.4 mmol, 2 eq) was added thereto, and the reaction mixture was stirred at room temperature for four hours. The reaction solution was cooled again in an ice bath, 200 mL of distilled water was added thereto, and extraction was performed twice with ethyl acetate. The combined organic layer was washed with brine and then dried over anhydrous magnesium sulfate. The organic layer was concentrated with a rotary evaporator to obtain 19.5 g of a crude product. The obtained crude product was purified by silica gel column chromatography (eluent, hexane: ethyl acetate = gradient from 2:1 to 1:1) and recrystallization with a mixed solution of hexane and ethyl acetate to obtain 14.0 g of the compound 3 (37.2 mmol, yield 89%, light yellow crystal). The $^{1}$H-NMR data of the obtained compound 3 are shown below.

$^{1}$H NMR (400 MHz, $CDCl_3$, TMS as internal standard): $\delta$ 7.70 (dt, J = 8.3, 1.5 Hz, 2 H), 7.50 (t, J = 7.9 Hz, 1 H), 7.36-7.32 (m, 2 H), 7.29-7.25 (m, 1 H, overlapped with the signal of residual $CHCl_3$), 7.22 (dd, J = 8.3, 7.3 Hz, 1 H), 7.13-7.08 (m,1 H), 7.06-6.97 (m, 3 H), 3.76-3.65 (m, 1 H), 3.52-3.31 (m, 6 H), 1.28 (t, J = 7.2 Hz, 3 H), 1.18 (t, J = 7.2 Hz, 3 H).

[Example 3]

<Synthesis of compound 4>

A compound 4 shown below was synthesized by a method described below according to the following reaction formula.

[Chem. 35]

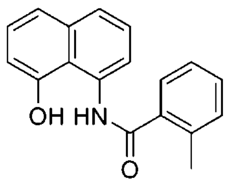

(Compound 4)

[Chem. 36]

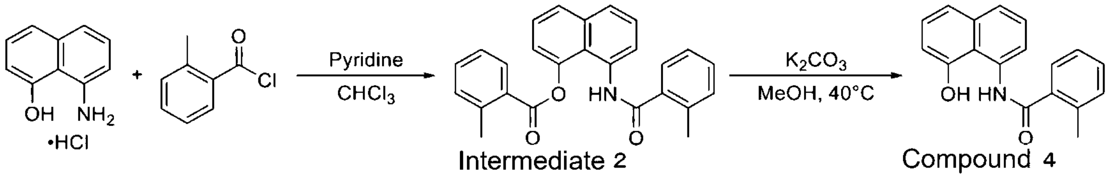


Intermediate 2

Compound 4

2.93 g of 8-amino-1-naphthol hydrochloride (15.0 mmol, 1 eq) and 60 mL of chloroform (dehydrated) were added into an oven dried 300 mL three-necked flask containing a heated and magnetic stirring bar in a nitrogen atmosphere. After that, 5.93 g of pyridine (dehydrated) (75.0 mmol, 5 eq) was added dropwise thereto at room temperature. After the reaction solution was cooled in an ice bath, 5.52 g of o-toluoyl chloride (35.7 mmol, 2.4 eq) was added dropwise thereto. After the end of the dropwise addition, the reaction solution was stirred at room temperature for 20 hours. After the end of a reaction, the reaction solution was cooled again in the ice bath, 15 mL of methanol was added thereto, and the reaction solution was stirred at room temperature for 30 minutes. Water and dichloromethane were added thereto, and extraction was performed four times with the dichloromethane. A combined organic layer was dried over sodium sulfate and then concentrated with a rotary evaporator. 9.00 g of an obtained crude product was purified by silica gel column chromatography (eluent, hexane: ethyl acetate = gradient from 95:5 to 90:10) to obtain 5.03 g of an intermediate 2. 5.03 g of the intermediate 2, 100 mL of methanol, and 2.29 g of potassium carbonate (16.6 mmol) were added into a 300 mL round-bottom flask, and the solution were stirred at 40°C for two hours. After the end of a reaction, water and ethyl acetate were added thereto, extraction was performed four times with ethyl acetate, and extraction was further performed twice with dichloromethane. The combined organic layer was dried over sodium sulfate and then concentrated with the rotary evaporator to obtain a brown solid crude product. Furthermore, the reaction was performed again on a third scale to obtain a crude product. The crude products of both reactions were combined together, ultrasonic-washed with hexane, and filtered to obtain 4.54 g (16.4 mmol) of the compound 4. The $^1$H-NMR data of the compound 4 obtained are shown below.

$^1$H-NMR (400 MHz, $CDCl_3$, TMS as internal standard): δ 2.53 (s, 3 H), 6.75 (dd, J = 1.0, 7.6 Hz, 1 H), 7.18-7.27 (m, 4 H, overlapped with the signal of residual $CHCl_3$), 7.33 (td, J = 1.3, 7.4 Hz, 1 H), 7.40-7.49 (m, 2 H), 7.52-7.60 (m, 2 H), 8.82 (d, J = 7.3 Hz, 1H), 11.00 (s, 1 H).

<Synthesis of compound 5>

A compound 5 shown below was synthesized by a method described below according to the following reaction formula.

[Chem. 37]

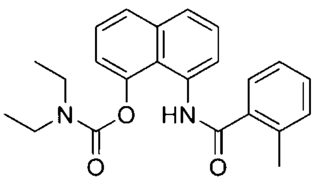

(Compound 5)

[Chem. 38]

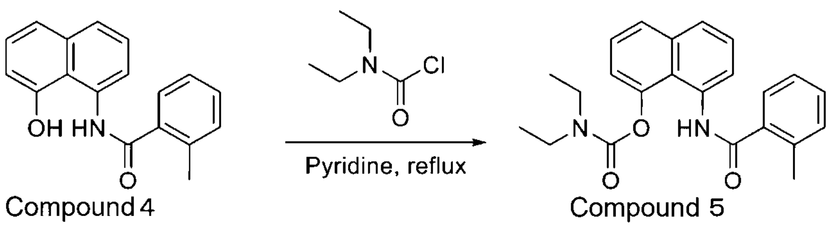


Compound 4

Compound 5

3.19 g of the compound 4 (11.5 mmol, 1 eq) and 60 mL of pyridine (dehydrated) were added into an oven dried 300 mL three-necked flask containing a heated and magnetic stirring bar in a nitrogen atmosphere. After that, 3.90 mL of

diethylcarbamoyl chloride (30.8 mmol, 2.7 eq) was added dropwise thereto at room temperature, and the solution were refluxed for 16 hours. After the end of a reaction, the reaction solution was cooled in an ice bath, 30 mL of water was added thereto, and the reaction solution was stirred at room temperature for 30 minutes. Water and ethyl acetate were added thereto, and extraction was performed three times with ethyl acetate. A combined organic layer was washed with water three times, further washed with brine once, dried over sodium sulfate, and then concentrated with a rotary evaporator. 4.61 g of the obtained crude product was purified by silica gel column chromatography (eluent, eluent, hexane: ethyl acetate = gradient from 90:10 to 80:20) to obtain 3.51 g of the compound 5 (9.32 mmol, yield 81%). The $^1$H-NMR data of the compound 5 obtained are shown below.

$^1$H NMR (400 MHz, $CDCl_3$, TMS as internal standard): δ 0.93-1.06 (m, 6 H), 2.52 (s, 3 H), 2.80-2.99 (m, 2 H), 3.15-3.26 (m, 2 H), 7.05 (br d, J = 7.6 Hz, 1 H), 7.18-7.29 (m, 2 H, overlapped with the signal of residual $CHCl_3$), 7.32-7.38 (m, 1 H), 7.39-7.44 (m, 1 H), 7.47-7.57 (m, 2 H), 7.66 (dd, J = 0.9, 8.2 Hz, 1 H), 7.72 (dd, J = 0.9, 8.2 Hz, 1 H), 8.66 (m, 1 H), 9.52 (br s, 1 H).

[Example 4]

<Synthesis of compound 6>

A compound 6 shown below was synthesized by a method described below according to the following reaction formula.

[Chem. 39]

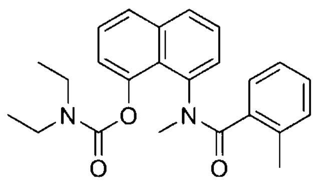

(Compound 6)

[Chem. 40]

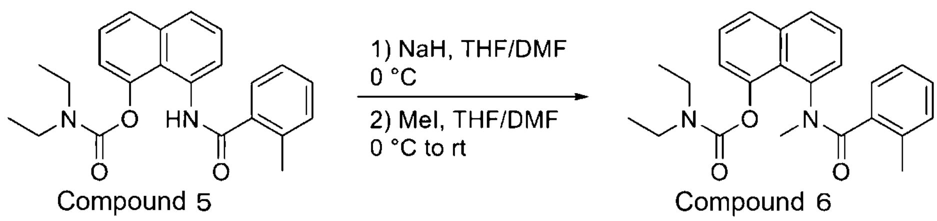


2.45 g of the compound 5 (6.50 mmol, 1 eq), 36 mL of tetrahydrofuran (dehydrated), and 4 mL of N,N'-dimethylformamide (dehydrated) were added into an oven dried 200 mL three-necked flask containing a heated and magnetic stirring bar in a nitrogen atmosphere. After the reaction solution was cooled in an ice bath, 0.37 g of sodium hydride (55%, liquid paraffin dispersion, 8.50 mmol, 1.3 eq) was added thereto. After the reaction solution was stirred for 50 minutes, 1.11 mL of iodomethane (17.8 mmol, 2.7 eq) was added dropwise thereto. The reaction solution was stirred for 20 minutes, then heated to room temperature, and further stirred for 18.5 hours. After the end of a reaction, the reaction solution was cooled again in the ice bath, 10 mL of water was added thereto, and the reaction solution was stirred at room temperature for 30 minutes. Water and ethyl acetate were added thereto, and extraction was performed three times with ethyl acetate. A combined organic layer was washed with water three times, further washed with brine once, dried over sodium sulfate, and then concentrated with a rotary evaporator, thereby obtaining 3.13 g of a crude product. Together with 1.74 g of a crude product obtained by separately performing the same reaction as described above on a small scale, the crude product was purified by silica gel column chromatography (eluent, hexane: ethyl acetate = gradient from 80:20 to 70:30) to obtain 3.75 g of the compound 6 (9.16 mmol). The $^1$H-NMR data of the compound 6 obtained are shown below.

$^1$H-NMR (400 MHz, $CDCl_3$, TMS as internal standard): δ 1.29 (dt, J = 7.2, 25.1 Hz, 6 H), 2.46 (s, 3 H), 3.33-3.44 (m, 5 H), 3.58 (m, 1 H), 3.86 (m, 1 H), 6.62-6.67 (m, 1 H), 6.91 (td, J = 7.3, 8.3 Hz, 1 H), 6.98-7.05 (m, 3 H), 7.17 (dd, J = 7.3, 8.3 Hz, 1 H), 7.29 (dd, J = 1.1, 7.7 Hz, 1 H), 7.48 (t, J = 7.9 Hz, 1 H), 7.63-7.68 (m, 2 H).

[Example 5]

<Synthesis of compound 7>

A compound 7 shown below was synthesized by a method described below according to the following reaction formula.

[Chem. 41]

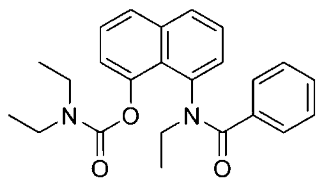

(Compound 7)

[Chem. 42]

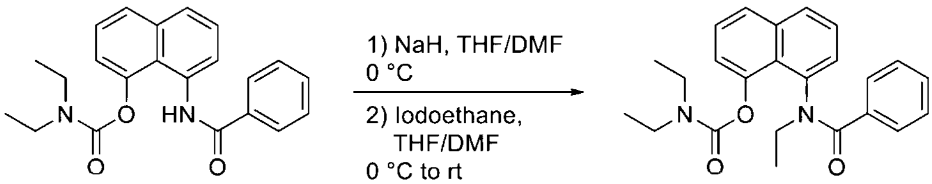


Compound 2 Compound 7

2.89 g of the compound 2 (7.9 mmol, 1 eq), 45 mL of tetrahydrofuran (dehydrated), and 9 mL of N,N-dimethylformamide (dehydrated) were added into an oven dried 200 mL three-necked flask containing a heated and magnetic stirring bar in a nitrogen atmosphere. After that, the solution were cooled in an ice bath, 0.44 g of sodium hydride (55%, liquid paraffin dispersion, 10.0 mmol, 1.3 eq) was then slowly added thereto, and the solution were stirred for 30 minutes. After that, 1.36 g of iodomethane (8.7 mmol, 1.1 eq) was added dropwise thereto. After the end of the dropwise addition, the reaction solution was heated to room temperature and stirred for 19 hours. After the end of a reaction, the reaction solution was cooled again in an ice bath, 3 mL of water was added thereto, and the reaction solution was stirred at room temperature for 30 minutes. Water and ethyl acetate were added to the reaction solution, extraction was then performed once with ethyl acetate, and the organic phase was washed with water three times and washed with brine once. A combined organic layer was dried over magnesium sulfate and concentrated with a rotary evaporator. The obtained crude product was purified by silica gel column chromatography (developing solvent eluent, hexane: ethyl acetate = gradient from 100:0 to 70:30) and ODS column chromatography (water and methanol were used as a developing solvent eluent, water: methanol = gradient from 100:0 to 55:45) to obtain 1.59 g (4.1 mmol, yield 51%, white crystal) of the compound 7. The $^1$H-NMR data of the compound 7 obtained are shown below.

$^1$H-NMR (400 MHz, $CDCl_3$, TMS as internal standard): $\delta$ 7.74-7.69 (m, 2 H), 7.52 (t, J = 7.9 Hz, 1 H), 7.35-7.29 (m, 3 H), 7.19 (dd, J = 8.3, 7.3 Hz, 1 H), 7.11 (tt, J = 7.3, 1.7 Hz, 1 H), 7.03 (tt, J = 7.4, 1.5 Hz, 2 H), 6.87 (dd, J = 7.3, 1.2 Hz, 1 H), 4.58 (td, J = 13.7, 6.7 Hz, 1 H), 3.69 (td, J = 14.4, 7.1 Hz, 1 H), 3.51-3.33 (m, 3 H), 3.23 (dt, J = 20.3, 7.0 Hz, 1 H), 1.25 (t, J = 7.2 Hz, 3 H), 1.18 (td, J = 7.0, 4.0 Hz, 6 H).

[Example 6]

<Synthesis of compound 8>

A compound 8 shown below was synthesized by a method described below according to the following reaction formula.

[Chem. 43]

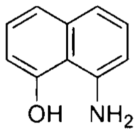

(Compound 8)

[Chem. 44]

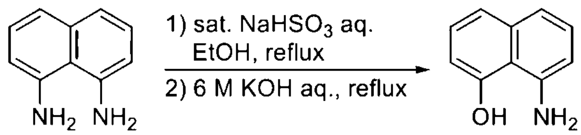


51.4 g of 1,8-diaminonaphthalene (324.6 mmol) and 171.2 mL of ethanol were added into an oven dried 2 L three-necked flask containing a heated and magnetic stirring bar in a nitrogen atmosphere and stirred at room temperature. Next, 342.4 mL of a saturated sodium hydrogen sulfite aqueous solution was added dropwise thereto at room temperature. After the end of the dropwise addition, the solution were heated and reflexed for 24 hours. After that, the solution were cooled to room temperature, 171.2 mL of a potassium hydroxide aqueous solution (6 mol/L) was added thereto, and the solution were heated and reflexed for 3.5 hours. After that, the solution were cooled to room temperature, and 2.5 L of hydrochloric acid (2 mol/L) was added thereto. An obtained aqueous solution was removed, this was extracted with 500 mL of ethyl acetate three times, and the target compound was extracted into a water layer from a combined organic layer using 500 mL of hydrochloric acid (1 mol/L). 300 mL of a saturated sodium carbonate aqueous solution was added to the water layer, and extraction was performed with 500 mL of ethyl acetate. A combined organic layer was dried over magnesium sulfate and concentrated with a rotary evaporator to obtain 33.81 g (yield 65%) of 8-amino-1-naphthol.

<Synthesis of compound 9>

A compound 9 shown below was synthesized by a method described below according to the following reaction formula.

[Chem. 45]

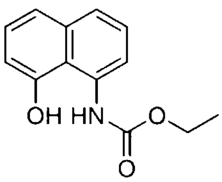

(Compound 9)

[Chem. 46]

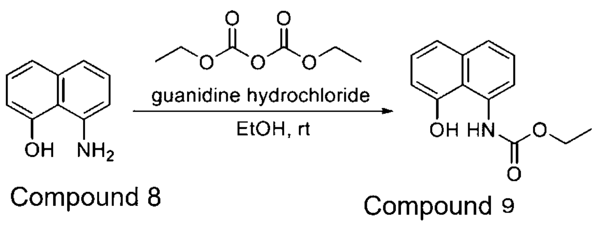


Compound 8 Compound 9

15.6 g of the compound 8 (98.1 mmol, 1 eq) and 156 mL of ethanol were added into an oven dried 1 L three-necked flask containing a heated and magnetic stirring bar in a nitrogen atmosphere and stirred while being cooled in a water bath. Next, 21.3 mL of diethyl dicarbonate (147.2 mmol, 1.5 eq) and 1.41 g of guanidine hydrochloride (14.7 mmol, 0.15 eq) were added thereto, and the solution were stirred at room temperature for 1.5 hours. The reaction solution was

concentrated, and 300 mL of distilled water and 300 mL of ethyl acetate were added to the obtained residue. Extraction was performed three times with ethyl acetate, and a combined organic layer was dried over magnesium sulfate and concentrated with a rotary evaporator. The obtained crude product was purified by two times of silica gel column chromatography (an eluent in the first time: chloroform/methanol, an eluent in the second time: hexane/ethyl acetate) to obtain 16.2 g of the compound 9 (70.1 mmol, yield 71%).

<Synthesis of compound 10>

A compound 10 shown below was synthesized by a method described below according to the following reaction formula.

[Chem. 47]

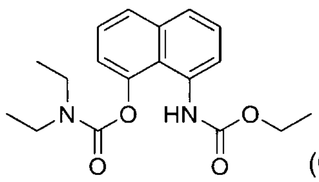

(Compound 10)

[Chem. 48]

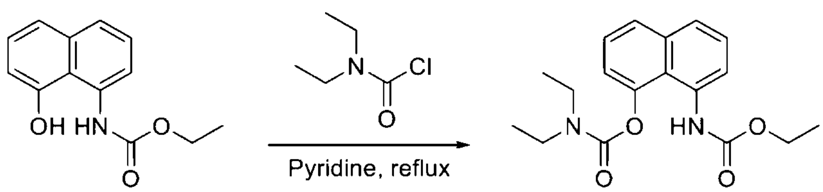


Compound 9 Compound 10

16.2 g of the compound 9 (69.9 mmol, 1 eq) and 300 mL of pyridine (dehydrated product) were added into an oven dried 1 L three-necked flask containing magnetic stirring bar in a nitrogen atmosphere, 9.30 mL of N,N-diethylcarbamoyl chloride (73.4 mmol, 1.05 eq) was then added thereto, and the temperature was raised to 120°C to heat and reflux the solution. After four hours, the reaction solution was cooled to room temperature, and 500 mL of distilled water was added thereto. A water layer was extracted with diethyl ether three times, the combined organic layer was washed with each of hydrochloric acid (1 mol/L) and a saturated sodium bicarbonate aqueous solution in order once, and the organic layer was then dried over anhydrous magnesium sulfate and concentrated with a rotary evaporator. The obtained crude product was purified by two times of silica gel column chromatography (eluent in the first time: chloroform/methanol, eluent in the second time: hexane/ethyl acetate) and further washed using a mixed solvent of hexane and ethyl acetate in a mixing ratio of 9:1 to obtain 13.95 g of the compound 10 (yield 60%). The $^1$H-NMR data of the compound 10 obtained are shown below.

$^1$H-NMR (500 MHz, $CDCl_3$, TMS as internal standard): δ 1.25 (t, J = 7.5 Hz, 3 H), 1.31 (t, J = 7.0 Hz, 3 H), 1.37 (t, J = 7.5 Hz, 3 H), 3.46 (q, J = 7.5 Hz, 2 H), 3.61 (q, J = 7.5 Hz, 2 H), 4.23 (q, J = 7.0 Hz, 2 H), 7.08-7.10 (m, 1 H), 7.39-7.46 (m, 2 H), 7.57-7.58 (m, 1 H), 7.68-7.70 (m, 1 H), 8.20 (br s, 1 H), 8.50 (br s, 1 H).

[Example 7]

<Synthesis of compound 11>

A compound 11 shown below was synthesized by a method described below according to the following reaction formula.

[Chem. 49]

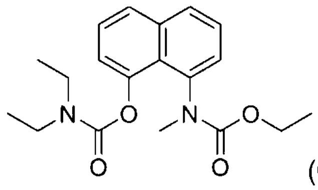

(Compound 11)

[Chem. 50]

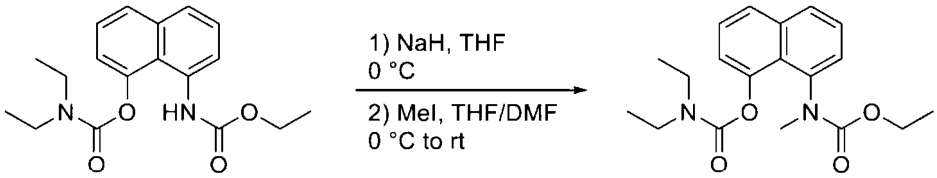


Compound 10 Compound 11

21.6 g of the compound 10 (65.5 mmol) and 655 mL of tetrahydrofuran (dehydrated) were added into an oven dried 3 L three-necked flask containing a heated and magnetic stirring bar in a nitrogen atmosphere, and the reaction solution was cooled in an ice bath. 3.43 g of sodium hydride (55%, liquid paraffine-dispersed product, 78.6 mmol, 1.2 eq) was slowly added thereto, and after one hour, 4.28 mL of iodomethane (67.8 mmol, 1.05 eq) was added dropwise thereto. After the end of the dropwise addition, the reaction solution was heated to room temperature and stirred at room temperature for one hour. After the reaction solution was cooled in an ice bath again, 300 mL of distilled water was added dropwise thereto. The reaction solution was extracted with ethyl acetate three times, and the combined organic layer was washed with brine, then dried over magnesium sulfate, and concentrated with a rotary evaporator. The obtained crude product was purified by multiple rounds of silica gel column chromatography to obtain 5.35 g of the compound 11 (yield 24%).

$^{1}$H NMR (500 MHz, $CDCl_3$, TMS as internal standard) : $\delta$ 0.98-1.37 (m, 9 H), 3.23-4.29 (m, 9 H), 7.12-7.14 (m, 1 H), 7.26-7.34 (m, 1 H, overlapped with the signal of residual $CHCl_3$), 7.42-7.47 (m, 2 H), 7.74-7.75 (m, 1 H), 7.80-7.82 (m, 1 H).

* Since a rotamer was contained, an integral value of signals derived from protons at the same places in the structure was added up to obtain an integer value.

[Example 8]

<Synthesis of compound 12>

A compound 12 shown below was synthesized by a method described below according to the following reaction formula.

[Chem. 51]

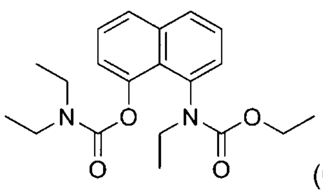

(Compound 12)

[Chem. 52]

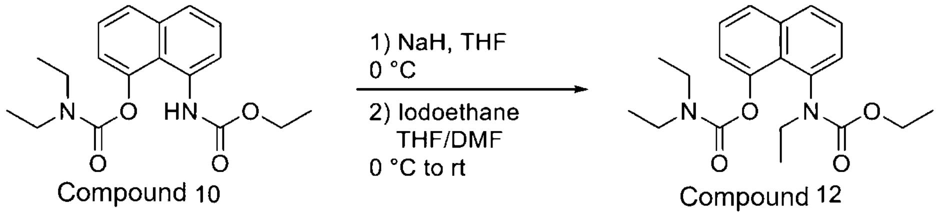


Compound 10 Compound 12

3.61 g of the compound 10 (10.9 mmol, 1 eq) and 54 mL of tetrahydrofuran (dehydrated) were added into an oven dried 200 mL three-necked flask containing a heated and magnetic stirring bar in a nitrogen atmosphere. After that, the solution were cooled in an ice bath, 0.50 g of sodium hydride (55%, liquid paraffin dispersion, 11.6 mmol, 1.1 eq) was then slowly added thereto, and the solution were stirred for 30 minutes. After that, 1.88 g of iodomethane (12.0 mmol, 1.1 eq) was slowly added dropwise thereto. After the end of the dropwise addition, the solution were heated to room temperature and stirred for 20 hours. After the end of a reaction, the reaction solution was cooled again in an ice bath, 0.53 g of ethanol (11.6 mmol, 1.1 eq) was added thereto, and the solution were stirred at room temperature for 30 minutes. The reaction solution was concentrated with a rotary evaporator. The obtained crude product was purified by silica gel column chromatography (eluent, hexane: ethyl acetate = gradient from 100:0 to 76:24) and then purified by recrystallization with a mixed solvent of hexane and dichloromethane to obtain 2.57 g (7.18 mmol, yield 66%, white crystal) of the compound 12. The $^1$H-NMR data of the compound 12 obtained are shown below.

$^1$H-NMR (400 MHz, $CDCl_3$, TMS as internal standard): δ 7.83 (dd, J = 8.3, 1.0 Hz, 1 H), 7.74 (dd, J = 8.3,1.0 Hz, 1 H), 7.44 (q, J = 7.6 Hz, 2 H), 7.23 (dd, J = 7.3, 1.2 Hz, 1 H), 7.13 (dd, J = 7.6, 1.0 Hz, 1 H), 4.16-3.96 (m, 3 H), 3.79-3.17 (m, 5 H), 1.28 (t, J = 7.2 Hz, 3 H), 1.22 (t, J = 7.1 Hz, 3 H), 1.17 (t, J = 7.2 Hz, 3 H), 1.01 (t, J = 7.2 Hz, 3 H).

[Example 9]

<Synthesis of imidazolium salt 1>

An imidazolium salt 1 shown below was synthesized in accordance with the following reaction formula by a method described below for the purpose of using the imidazolium salt 1 for the synthesis of a compound 13 described below.

[Chem. 53]

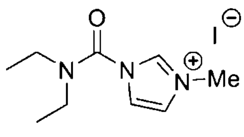

(Imidazolium salt 1)

[Chem. 54]

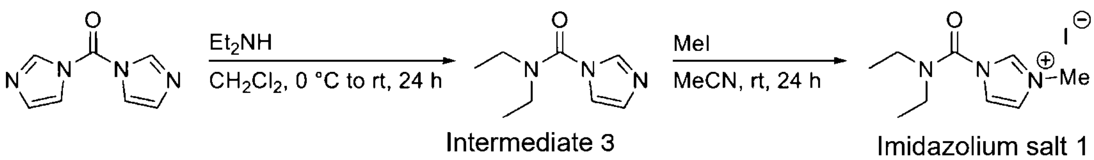


Intermediate 3 Imidazolium salt 1

53.5 g of carbonylimidazole (330 mmol, 1.1 eq) and 200 mL of dichloromethane (dehydrated) were added into an oven dried 500 mL three-necked flask containing a heated and magnetic stirring bar in a nitrogen atmosphere, and the reaction solution was cooled in an ice bath. After the cooling, 21.9 g of diethylamine (300 mmol, 1 eq) was added dropwise thereto over 20 minutes. After the end of the dropwise addition, the reaction solution was slowly heated up to room temperature and stirred for 24 hours while being maintained at room temperature. The reaction solution was cooled in an ice bath again, and 200 mL of water was added dropwise thereto. Extraction was performed four times with dichloromethane, the combined organic layer was dried over sodium sulfate and concentrated with a rotary evaporator to obtain 56.3 g of an intermediate 3 with a purity of 78%.

The intermediate 3 obtained by the above-described method was transferred to a 1 L reaction container, the inside was substituted with nitrogen, 500 mL of acetonitrile (dehydrated) and 181.4 g of iodomethane (1.28 mol, 4.9 eq) were added thereto, and the solution were stirred at room temperature for 24 hours. After that, the solution were concentrated in their entirety to obtain 111.7 g of the imidazolium salt 1 with a purity of 81.3%.

<Synthesis of compound 13>

A compound 13 shown below was synthesized by a method described below according to the following reaction formula.

[Chem. 55]

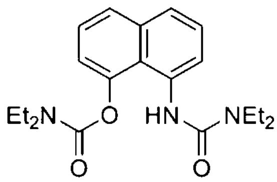

(Compound 13)

[Chem. 56]

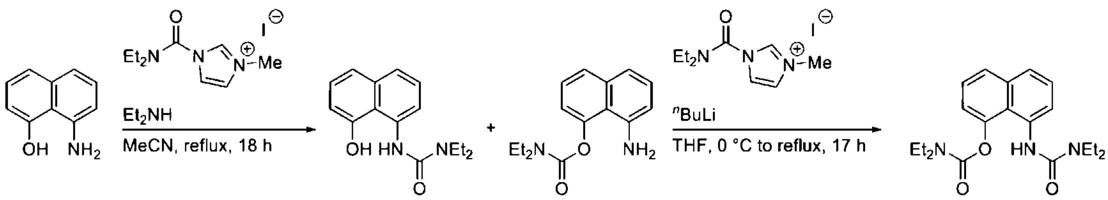


Compound 8

Intermediate 4

Compound 13

52.5 g of the imidazolium salt 1 (138 mmol, 1.1 eq), 500 mL of acetonitrile (dehydrated), 19.9 g of the compound 8 (125 mmol, 1 eq), and 15.2 g of triethylamine (150 mmol, 1.2 eq) were sequentially added into an oven dried 1 L four-necked flask containing a heated and magnetic stirring bar in a nitrogen atmosphere. The temperature was raised until the internal temperature of the reaction solution reached 78.5°C, and the reaction solution was heated and refluxed for 18 hours. After that, the reaction solution was cooled to room temperature and then concentrated. 500 mL of dichloromethane was added to an obtained black oil, and a generated solid was filtered. 400 mL of water was added to a filtrate, and extraction was performed four times with dichloromethane. The combined organic layer was washed with water three times, further washed with brine once, then dried over sodium sulfate, and concentrated with a rotary evaporator. The obtained crude product was purified by silica gel column chromatography (hexane: ethyl acetate = 50:50) to obtain 29.5 g of a mixture containing the intermediate 4.

27.4 g of the mixture containing the intermediate 4 (NMR purity 75%, 80 mmol, 1 eq) and 500 mL of tetrahydrofuran (dehydrated) were added into an oven dried 1 L four-necked flask equipped with a magnetic stirrer in a nitrogen atmosphere and cooled in an ice bath. After that, 56 mL of $^n$BuLi (hexane solution, 1.57 mol/L, 88 mmol, 1.1 eq) was added dropwise thereto over 20 minutes. After the end of the dropwise addition, the temperature was raised until the internal temperature reached 65°C, and the reaction solution was heated and refluxed for 17 hours as it was. After that, the reaction solution was cooled in an ice bath, and 100 mL of water was added thereto. Extraction was performed three times with ethyl acetate, and the combined organic layer was washed with water three times and further washed with brine once. The organic layer was dried over sodium sulfate and then concentrated with the rotary evaporator. The obtained crude product was purified by multiple rounds of silica gel column chromatography to obtain 5.33 g of the compound 13 (yield 19%).

[Example 10]

<Synthesis of compound 14>

A compound 14 shown below was synthesized by a method described below according to the following reaction formula.

[Chem. 57]

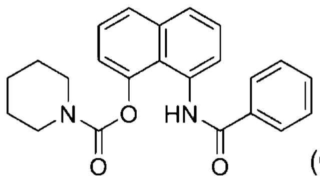
(Compound 14)

[Chem. 58]

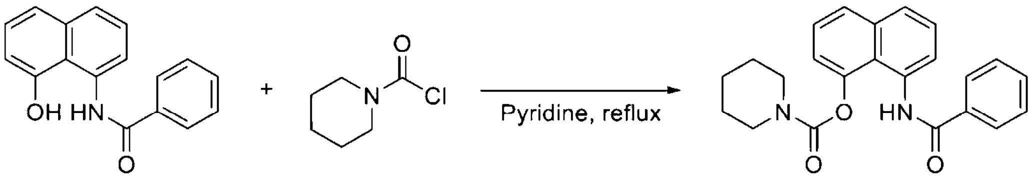

Compound 1

Compound 14

3.03 g of the compound 1 (11.5 mmol, 1 eq) and 60 mL of pyridine (dehydrated) were added into an oven dried 300 mL three-necked flask containing a heated and magnetic stirring bar in a nitrogen atmosphere. After that, 3.63 mL of 1-piperidinecarbonyl chloride (28.8 mmol, 2.5 eq) was slowly added dropwise thereto at room temperature, and the solution were heated and refluxed for 26.5 hours (oil bath 115°C, internal temperature 115°C). After the end of a reaction, the reaction solution was cooled in an ice bath, 30 mL of water was slowly added thereto, and the reaction solution was stirred at room temperature for 30 minutes. After water and ethyl acetate were added thereto, extraction was performed twice with ethyl acetate, and extraction was further performed twice with dichloromethane. The combined organic layer was washed with water three times, sodium bicarbonate was added to a water layer, and extraction was performed three times with dichloromethane. The combined organic layer was dried over sodium sulfate and then concentrated with a rotary evaporator. 5.40 g of the obtained crude product was purified by silica gel column chromatography (eluent, hexane: dichloromethane = gradient from 50:50 to 0:100) to obtain 3.43 g of the compound 14 (9.16 mmol, yield 80%). The $^{1}$H-NMR data of the compound 14 obtained are shown below.

$^{1}$H NMR (400 MHz, $CDCl_3$, TMS as internal standard): δ 1.33 (br s, 2 H), 1.48 (br s, 4 H), 3.28 (m, 4 H), 7.09 (dd, J = 1.2, 7.6 Hz, 1 H), 7.32-7.45 (m, 1 H), 7.48-7.60 (m, 4 H), 7.70 (dd, J = 1.0, 8.3 Hz, 1 H), 7.74 (dd, J = 1.0, 8.3 Hz, 1 H), 7.88-7.93 (m, 2 H), 8.32 (br d, J = 6.8 Hz, 1 H), 9.41 (br s, 1 H).

[Example 11]

<Synthesis of compound 15>

A compound 15 shown below was synthesized by a method described below according to the following reaction formula.

[Chem. 59]

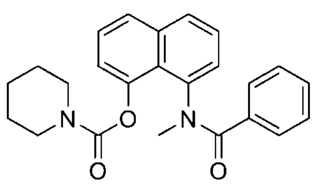
(Compound 15)

[Chem. 60]

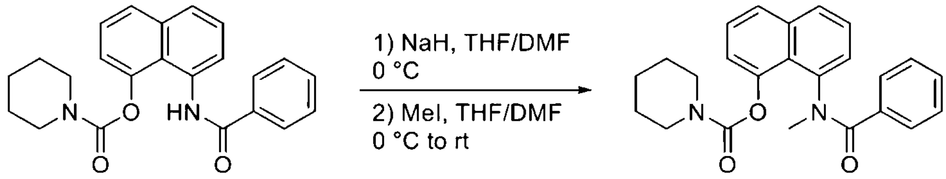


Compound 14 Compound 15

2.32 g of the compound 14 (6.20 mmol, 1 eq), 36 mL of tetrahydrofuran (dehydrated), and 4 mL of N,N'-dimethylformamide (dehydrated) were added into an oven dried 200 mL three-necked flask including a magnetic stirring bar in a nitrogen atmosphere. After the reaction solution was cooled in an ice bath, 0.35 g of sodium hydride (55%, liquid paraffin-dispersed product, 8.10 mmol, 1.3 eq) was slowly added thereto. After the reaction solution was stirred for 40 minutes, 0.46 mL of iodomethane (7.40 mmol, 1.2 eq) was slowly added dropwise thereto. The reaction solution was stirred for 20 minutes, then slowly heated to room temperature, and further stirred for 19 hours. After the end of a reaction, the reaction solution was cooled again in an ice bath, 10 mL of water was slowly added thereto, and the reaction solution was stirred at room temperature for 30 minutes. Water and ethyl acetate were added thereto, and extraction was performed three times with ethyl acetate. A combined organic layer was washed with water three times, further washed with brine once, dried over sodium sulfate, and then concentrated with a rotary evaporator. As a result of purifying 2.77 g of the obtained crude product by silica gel column chromatography (eluent, hexane: ethyl acetate = gradient from 85:15 to 0:100) to obtain 2.26 g of the compound 15 (6.20 mmol, yield 94%). The $^1$H-NMR data of the compound 15 obtained are shown below.

$^1$H NMR (400 MHz, $CDCl_3$, TMS as internal standard): δ 1.52-1.81 (m, 6 H), 3.39-3.79 (m, 7 H), 6.98 (dd, J = 1.2, 7.3 Hz, 1 H), 7,00-7.06 (m, 2 H), 7.08-7.14 (m, 1 H), 7.21 (dd, J = 7.3, 8.0 Hz, 1 H), 7.28 (dd, J = 1.1, 7.7 Hz, 1 H), 7.32-7.36 (m, 2 H), 7.50 (t, J = 7.9 Hz, 1 H), 7.71 (dd, J = 3.5, 7.2 Hz, 2 H).

[Example 12]

<Synthesis of compound 16>

A compound 16 shown below was synthesized by a method described below according to the following reaction formula.

[Chem. 61]

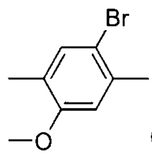

(Compound 16)

[Chem. 62]

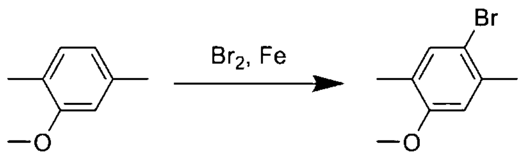


50.0 g of 2,5-dimethylanisole (367 mmol) and 0.65 g of an iron powder (11.6 mmol) were put into an oven dried 300 mL four-necked flask containing a heated and magnetic stirring bar, and 58.7 g of bromine (367 mmol) was added dropwise thereto at room temperature for 38 minutes while the solution were cooled in a water bath. The solution were further stirred at room temperature for two hours. The reaction solution was cooled in an ice bath, 200 mL of hexane and 100 mL of water were added thereto, a small amount of sodium thiosulfate was added thereto to change the color, and sodium hydroxide was added thereto to neutralize the reaction solution. The reaction solution was extracted with hexane, the combined organic layer was washed with brine, then dried over sodium sulfate, and concentrated with a rotary

evaporator. 84.4 g of the obtained crude product was vacuum-distilled to obtain 44.86 g of the compound 16 (yield 56%).

<Synthesis of compound 17>

A compound 17 shown below was synthesized by a method described below according to the following reaction formula.

[Chem. 63]

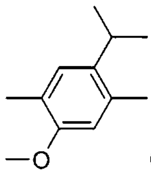

(Compound 17)

[Chem. 64]

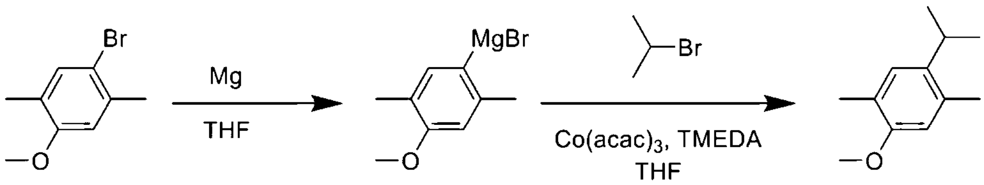


Compound 16 Intermediate 5 Compound 17

3.65 g of magnesium (150 mmol) was put into an oven dried 200 mL four-necked flask containing a heated and magnetic stirring bar, and heated and dried under reduced pressure, the inside was substituted with nitrogen, 40 mL of tetrahydrofuran was put thereinto, several drops of 1,2-dibromoethane was added thereto and heated, and gentle reflux was maintained while 22.09 g of the compound 16 (tetrahydrofuran solution, 100 mmol, 40 mL) was added dropwise thereto from a dripping funnel. The resultant was stirred until it reaches to room temperature to obtain a solution containing an intermediate 5.

1.78 g of cobalt (III) tris(acetylacetonate), 0.75 ml of tetramethyl ethylenediamine (5.00 mmol), and 14.0 ml of 2-bromopropane (149 mmol) were put into an oven dried 500 mL four-necked flask containing a heated and magnetic stirring bar in a nitrogen atmosphere and cooled in an ice bath. The solution of the intermediate 5 prepared by the procedure of the previous section was added dropwise thereto from a dripping funnel for 45 minutes. The solution were stirred for one hour and 20 minutes while remaining cooled in the ice bath, 1 mol/L of hydrochloric acid was then added dropwise thereto, and the reaction was stopped. The solution after the reaction was extracted with diethyl ether, the combined organic layer was washed with a sodium carbonate aqueous solution and brine, then dried over sodium sulfate, and concentrated with a rotary evaporator. 18.38 g of an obtained crude product was vacuum-distilled to obtain 15.9 g of the compound 17 with a yield of 86%.

<Synthesis of compound 18>

A compound 18 shown below was synthesized by a method described below according to the following reaction formula.

[Chem. 65]

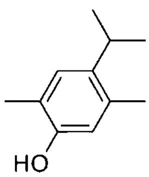

(Compound 18)

[Chem. 66]

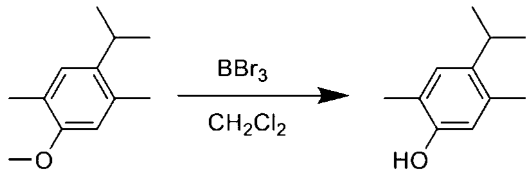


18.38 g of the compound 17 (93.9 mmol) and 125 ml of dichloromethane were put into an oven dried 500 mL four-necked flask containing a heated and magnetic stirring bar and cooled in an ice bath. While the solution were stirred, 100 mL of a dichloromethane solution of boron tribromide (concentration 1 mol/L) was added dropwise thereto from a dripping funnel for 30 minutes while the internal temperature was maintained at 3°C to 7°C. After that, the solution were stirred at room temperature for two hours, and water was then added dropwise thereto while the solution were cooled in the ice water bath. After that, extraction was performed with dichloromethane, the combined organic layer was washed with a sodium bicarbonate aqueous solution and brine, then dried over sodium sulfate, and concentrated with a rotary evaporator. 17.2 g of the obtained crude product was purified by silica gel column chromatography (using an eluent containing toluene and ethyl acetate in a mixing ratio of 100:1) to obtain 14.0 g of the compound 18 with a yield of 88%.

<Synthesis of compound 19>

A compound 19 shown below was synthesized by a method described below according to the following reaction formula.

[Chem. 67]

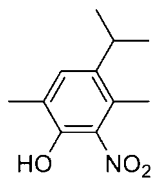
(Compound 19)

[Chem. 68]

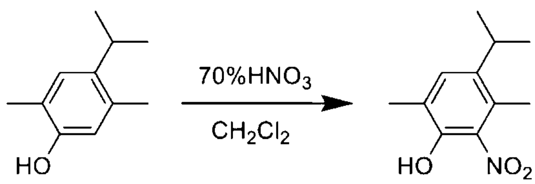


41.9 g of the compound 18 (249 mmol) and 290 ml of dichloromethane were put into an oven dried 1 L four-necked flask containing a heated and magnetic stirring bar and stirred while being cooled in an ice bath. 17.4 mL of nitric acid (70%, 274 mmol) was added dropwise thereto for 36 minutes while the internal temperature was maintained at 0.3°C to 2.3°C. Furthermore, the solution were stirred in the ice bath for 21 hours and subsequently stirred at room temperature for five hours, 300 mL of a sodium bicarbonate aqueous solution was then added thereto, and the organic layer was extracted with dichloromethane. The combined organic layer was dried over sodium sulfate and concentrated with a rotary evaporator, thereby obtaining 54.3 g of a crude product. The crude product was diluted with 100 mL of hexane and placed still at room temperature for 64 hours to remove precipitated crystals. A supernatant was purified by silica gel column chromatography (using a mixed solvent of hexane and acetone in a mixing ratio of 95:5) to obtain 17.69 g of the compound 19 with a yield of 33%.

<Synthesis of compound 20>

A compound 20 shown below was synthesized by a method described below according to the following reaction formula.

[Chem. 69]

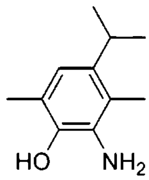

(Compound 20)

[Chem. 70]

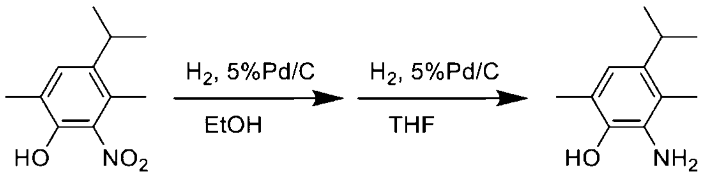


17.69 g of the compound 19 (82.4 mmol), 2.0 g of Pd/C (5%, 55% water), and 100 mL of ethanol were put into an oven dried 200 mL autoclave containing a heated and magnetic stirring bar, and the inside was substituted into a nitrogen atmosphere under stirring. The inside was pressurized such that the hydrogen pressure was maintained at 0.6 to 0.9 MPa while the solution were stirred at 50°C, the solution were stirred for 22 hours until a decrease in the hydrogen pressure was stopped, hydrogen was then discharged, and the inside was substituted into a nitrogen atmosphere. A precipitate in the container was dissolved by adding tetrahydrofuran thereto and filtered with a hydrophilic PTFE membrane filter having a pore diameter of 1 μm. A filtrate was concentrated with a rotary evaporator and put into an oven dried 200 mL autoclave containing a heated and magnetic stirring bar, furthermore, 100 mL of tetrahydrofuran and 1.0 g of Pd/C (5%, 55% water) were added thereto, and the inside was substituted into a nitrogen atmosphere under stirring. The inside was pressurized such that the hydrogen pressure was maintained at 0.6 to 0.9 MPa while the solution were stirred at 50°C, the solution were stirred for 4.5 hours until a decrease in the hydrogen pressure was stopped, hydrogen was then discharged, and the inside was substituted into a nitrogen atmosphere. The reaction solution was filtered with the hydrophilic PTFE membrane filter having a pore diameter of 1 μm, and a filtrate was concentrated with the rotary evaporator to obtain a solid crude product. This was washed with hexane and dried under reduced pressure to obtain 14.72 g of the compound 20 with a yield of 96%.

<Synthesis of compound 21>

A compound 21 shown below was synthesized by a method described below according to the following reaction formula.

[Chem. 71]

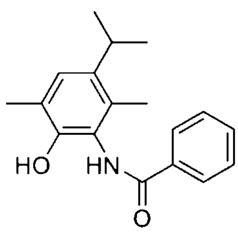

(Compound 21)

[Chem. 72]

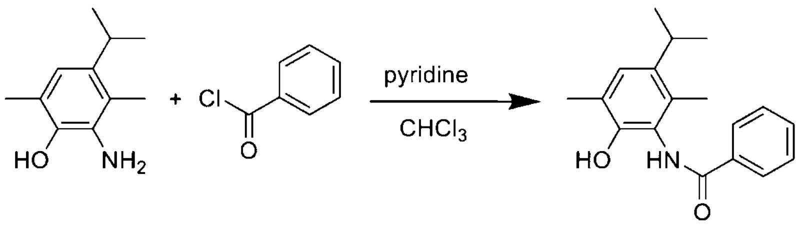


2.20 g of the compound 20 (12.3 mmol, 1 eq) and 65 mL of chloroform (dehydrated) were added into an oven dried 200 mL three-necked flask containing a heated and magnetic stirring bar in a nitrogen atmosphere. After that, the solution were cooled in an ice bath, 1.07 g of pyridine (dehydrated, 13.5 mmol, 1.1 eq) and 1.82 g of benzoyl chloride (12.9 mmol, 1.1 eq) were then slowly added thereto, and the solution were stirred for 10 minutes. After that, the ice bath was removed to raise the temperature to room temperature, and the solution were stirred for six hours. After the end of a reaction, the reaction solution was cooled again in an ice bath, 2.0 mL of methanol (49.3 mmol, 4 eq) was added thereto, and the reaction solution was stirred at room temperature for 30 minutes. Water and ethyl acetate were added to the reaction solution, and the reaction solution was extracted with ethyl acetate, washed with water three times, then further washed with brine once, dried over magnesium sulfate, and then concentrated with a rotary evaporator. 3.50 g of the obtained crude product was dissolved by adding 17 mL of dichloromethane thereto, and 70 mL of hexane was then added thereto under stirring to cool the crude product in an ice bath, thereby precipitating a solid. The obtained solid was filtered, collected, and washed with hexane three times to obtain 3.32 g of the compound 21 (yield 95%). The $^1$H-NMR data of the compound 21 obtained are shown below.

$^1$H-NMR (400 MHz, $CDCl_3$, TMS as internal standard): $\delta$ 7.95-7.92 (m, 2 H), 7.89 (s, 1 H), 7.81 (s, 1 H), 7.63-7.59 (m, 1 H), 7.56-7.51 (m, 2 H), 7.02 (s, 1 H), 3.16-3.06 (m, 1 H), 2.31 (d, J = 6.6 Hz, 6 H), 1.21 (d, J = 6.8 Hz, 6 H).

<Synthesis of compound 22>

A compound 22 shown below was synthesized by a method described below according to the following reaction formula.

[Chem. 73]

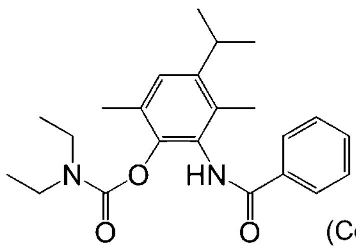
(Compound 22)

[Chem. 74]

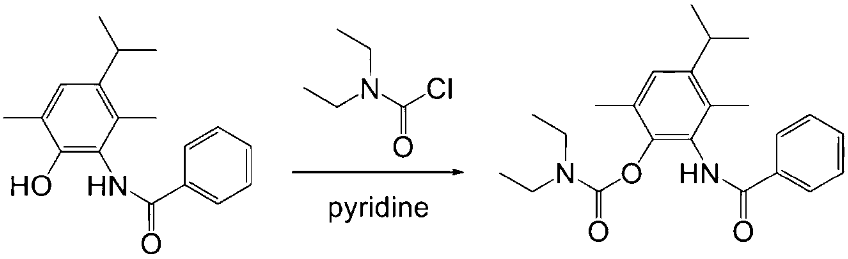


3.32 g of the compound 21 (11.7 mmol, 1 eq) and 40 mL of pyridine (dehydrated) were added into an oven dried 100 mL three-necked flask containing a heated and magnetic stirring bar in a nitrogen atmosphere. After that, 3.99 g of diethylcarbamoyl chloride (29.4 mmol, 2.5 eq) was added thereto while the solution were stirred at room temperature, and

the solution were refluxed for 17 hours in an oil bath at 120°C. After the end of a reaction, the reaction solution was cooled to room temperature and added to 400 mL of ice-cooled pure water. A precipitate was filtered with a Kiriyama funnel, washed with pure water three times, and then dried to obtain 3.32 g of a crude product. The obtained crude product was purified by silica gel column chromatography (eluent, hexane: ethyl acetate = gradient from 100:0 to 60:40) to obtain 1.35 g of the compound 22. The $^1$H-NMR data of the obtained compound 22 (3.5 mmol, yield 30%) are shown below.

$^1$H-NMR (400 MHz, $CDCl_3$, TMS as internal standard): δ 7.91-7.88 (m, 2 H), 7.80 (s, 1 H), 7.56-7.52 (m, 1 H), 7.49-7.44 (m, 2 H), 7.07 (s, 1 H), 3.37-3.30 (m, 4 H), 3.20-3.10 (m, 1 H), 2.23 (d, J = 2.2 Hz, 6 H), 1.22 (d, J = 6.8 Hz, 6 H), 1.07 (dt, J = 13.3, 5.9 Hz, 6 H).

[Example 13]

<Synthesis of compound 23>

A compound 23 shown below was synthesized by a method described below according to the following reaction formula.

[Chem. 75]

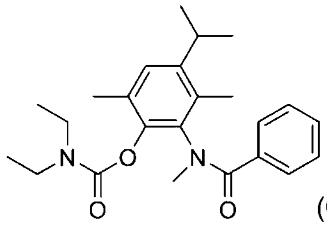

(Compound 23)

[Chem. 76]

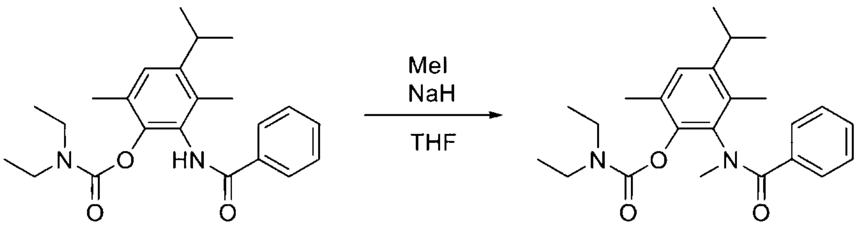


2.66 g of the compound 22 (6.9 mmol, 1 eq) and 45 mL of tetrahydrofuran (dehydrated) were added into an oven dried 200 mL three-necked flask containing a heated and magnetic stirring bar in a nitrogen atmosphere. After that, the solution were cooled in an ice bath, 0.32 g of sodium hydride (55%, liquid paraffin dispersion, 13.3 mmol, 1.9 eq) was then slowly added thereto, and the solution were stirred for 10 minutes. After that, 1.09 g of iodomethane (7.7 mmol, 1.1 eq) was slowly added dropwise thereto, and the solution were stirred for 100 minutes. After that, the solution were heated to room temperature and stirred for 18 hours. After the end of a reaction, the reaction solution was cooled again in the ice bath, 1 mL of methanol (24.7 mmol, 3.6 eq) was added thereto, and the reaction solution was stirred at room temperature for 10 minutes. After that, 10 mL of a saturated ammonium chloride aqueous solution was added thereto to stop the reaction. Pure water and ethyl acetate were added to the reaction solution, the reaction solution was extracted with ethyl acetate, washed with water twice, and washed with brine once, dried over magnesium sulfate, and then concentrated with a rotary evaporator. 2.97 g of the obtained crude product was suspended in 50 mL of hexane, and filtered and washed with hexane three times, thereby obtaining 2.28 g of a pale yellow crystal. The obtained crude product was purified by silica gel column chromatography (eluent, hexane: ethyl acetate = gradient from 100:0 to 80:20) to obtain 2.22 g of the compound 23 (5.60 mmol, yield 81%, white crystal). The $^1$H-NMR data of the compound 23 obtained are shown below.

$^1$H-NMR (400 MHz, $CDCl_3$, TMS as internal standard): δ 7.30 (brs, 2 H), 7.20 (tt, J = 7.3, 1.6 Hz, 1 H), 7.13-7.08 (m, 2 H), 6.95 (s, 1 H), 3.52-3.31 (m, 4 H), 3.27 (s, 3 H), 2.96 (brs, 1 H), 2.10 (brs, 6 H), 1.28 (t, J = 7.2 Hz, 3 H), 1.25-1.17 (m, 6 H), 1.01 (d, J = 5.6 Hz, 3 H).

[Example 14]

<Synthesis of compound 24>

A compound 24 shown below was synthesized by a method described below according to the following reaction formula.

[Chem. 77]

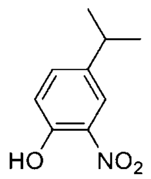

(Compound 24)

[Chem. 78]

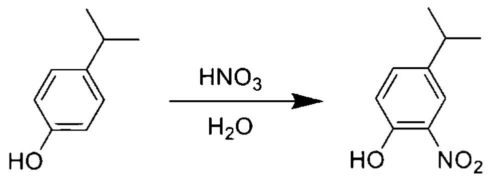


23.23 g of 4-isopropylphenyl (170.6 mmol) and 65.1 mL of water were put into an oven dried 500 mL three-necked flask containing a heated and magnetic stirring bar in a nitrogen atmosphere and cooled in an ice bath while being stirred. Next, 18.6 mL of nitric acid (67%, 280.8 mmol) was added dropwise thereto for 15 minutes while the internal temperature was maintained at 2.9°C or lower. After the solution were stirred for 1.5 hours, 580 mL of water was added thereto, and the reaction solution was extracted with ethyl acetate. The combined organic layer was dried over sodium sulfate and then concentrated with a rotary evaporator, thereby obtaining 30.23 g a crude product. The crude product was purified by silica gel column chromatography (eluent, hexane: dichloromethane = gradient from 4:1 to 2:1) to obtain 23.80 g of the compound 24 with a yield of 77%.

<Synthesis of compound 25>

A compound 25 shown below was synthesized by a method described below according to the following reaction formula.

[Chem. 79]

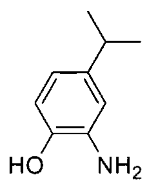

(Compound 25)

[Chem. 80]

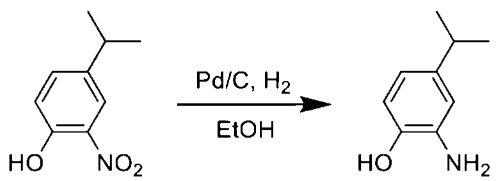


23.79 g of the compound 24 (131.3 mmol) and 703.9 ml of ethanol were put into an oven dried 2 L round-bottom flask containing a heated and magnetic stirring bar. Next, 2.35 g of Pd/C (10%, 55% water) was added thereto, the inside

was substituted with a hydrogen atmosphere by connecting a hydrogen balloon thereto, and the solution were stirred at room temperature for three hours. Th reaction solution was filtered, the filtrate was concentrated, hexane was added thereto, a solid obtained by the filtration was then washed with hexane and dried to obtain 20.38 g of the compound 25 with a yield of 93%.

<Synthesis of compound 26>

A compound 26 shown below was synthesized by a method described below according to the following reaction formula.

[Chem. 81]

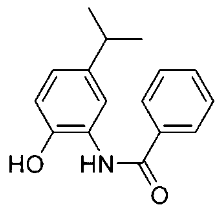
(Compound 26)

[Chem. 82]

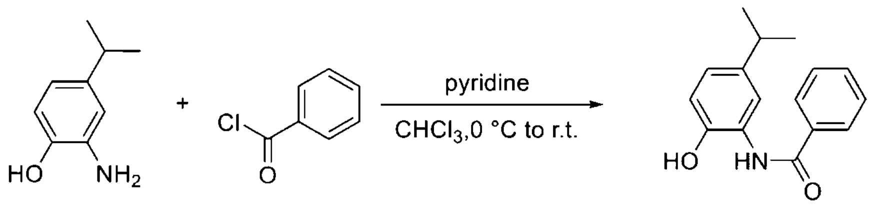


3.02 g of the compound 25 (20.0 mmol, 1 eq) and 100 mL of chloroform (dehydrated) were added into an oven dried 500 mL three-necked flask containing a heated and magnetic stirring bar in a nitrogen atmosphere. After that, 1.77 mL of pyridine (dehydrated, 22.0 mmol, 1.1 eq) was slowly added dropwise thereto at room temperature. After the reaction solution was cooled in an ice bath, 2.46 mL of benzoyl chloride (21.0 mmol, 1.05 eq) was slowly added dropwise thereto. After the end of the dropwise addition, the reaction solution was heated to room temperature and stirred for four hours. After the end of the reaction, the reaction solution was cooled again in an ice bath, 10 mL of methanol was added thereto, and the reaction solution was stirred. Water and dichloromethane were added thereto, and extraction was then performed three times with dichloromethane. The combined organic layer was washed with brine once, dried over sodium sulfate, and then concentrated with a rotary evaporator. 5.49 g of the obtained crude product was purified by silica gel column chromatography (eluent: dichloromethane 100%) in two separate runs to obtain 4.90 g (19.2 mmol, 96%) of the compound 26. The $^1$H-NMR data of the compound 26 obtained are shown below.

$^1$H-NMR (400 MHz, $CDCl_3$, TMS as internal standard): δ 8.43 (s, 1 H), 8.11 (br s, 1 H), 7.93-7.89 (m 2 H), 7.62-7.56 (m, 1 H), 7.54-7.48 (m, 2 H) 7.05-6.98 (m, 3 H), 2.92-2.79 (m, 1 H), 1.23 (d, J = 7.1 Hz, 6 H)

<Synthesis of compound 27>

A compound 27 shown below was synthesized by a method described below according to the following reaction formula.

[Chem. 83]

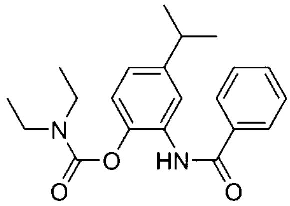
(Compound 27)

[Chem. 84]

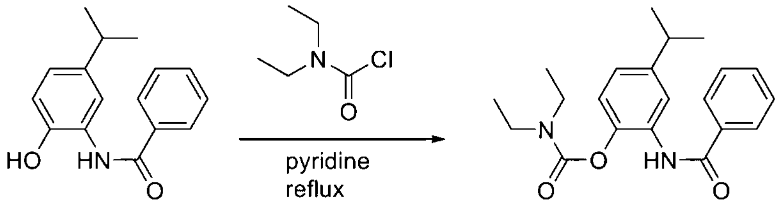


3.83 g of the compound 26 (15.0 mmol, 1 eq) and 100 mL of pyridine (dehydrated) were added into an oven dried 300 mL three-necked flask containing a heated and magnetic stirring bar in a nitrogen atmosphere. After that, 4.75 mL of diethylcarbamoyl chloride (37.5 mmol, 2.5 eq) was slowly added dropwise thereto at room temperature, and the solution were heated and refluxed for 22.5 hours. After the end of the reaction, the reaction solution was cooled in an ice bath, 30 mL of water was slowly added thereto, and the reaction solution was stirred at room temperature. After that, the reaction solution was concentrated with a rotary evaporator, water and ethyl acetate were added thereto, and the reaction solution was extracted with ethyl acetate three times. The combined organic layer was washed with water three times and with brine once, dried over sodium sulfate, and then concentrated with the rotary evaporator. 6.36 g of the obtained crude product was purified by silica gel column chromatography (eluent, hexane: ethyl acetate = gradient from 100:0 to 85:15) to obtain 4.90 g of the compound 27 (13.8 mmol, yield 92%). The $^1$H-NMR data of the compound 27 obtained are shown below.

1H-NMR (400 MHz, CDCl3, TMS as internal standard): δ 8.44 (br s, 1 H), 8.03 (br s, 1 H), 7.90-7.85 (m, 2 H), 7.54 (tt, J = 7.3, 1.7 Hz, 1 H), 7.50-7.45 (m, 2 H), 7.08-7.01 (m, 2 H), 3.50-3.35 (m, 4 H), 3.01-2.88 (m, 1 H), 1.30-1.17 (m, 12 H).

[Example 15]

<Synthesis of compound 28>

A compound 28 shown below was synthesized by a method described below according to the following reaction formula.

[Chem. 85]

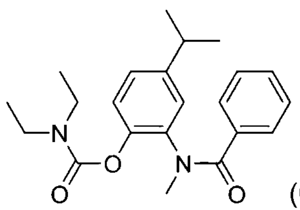
(Compound 28)

[Chem. 86]

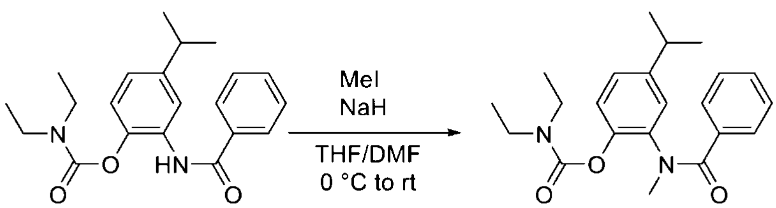

3.01 g of the compound 27 (8.50 mmol, 1 eq), 45 mL of tetrahydrofuran (dehydrated), and 5 mL of N,N'-dimethylformamide (dehydrated) were added into an oven dried 300 mL three-necked flask containing a heated and magnetic stirring bar in a nitrogen atmosphere. After the reaction solution was cooled in an ice bath, 0.48 g of sodium hydride (55%, liquid paraffin dispersion, 11.1 mmol, 1.3 eq) was slowly added thereto. After 30 minutes of stirring, 0.63 mL of iodomethane (10.2 mmol, 1.2 eq) was slowly added dropwise thereto. The reaction solution was slowly heated to room temperature and further stirred for 23 hours. After the end of the reaction, the reaction solution was cooled again in an ice bath, 10 mL of water was slowly added thereto, and the reaction solution was stirred at room temperature. Water and ethyl acetate were added thereto, and extraction was performed three times with ethyl acetate. The combined organic layer was washed with water three times and with brine once, dried over sodium sulfate, and then concentrated with a rotary evaporator. 3.43 g of the obtained crude product was purified by silica gel column chromatography (eluent, hexane: ethyl acetate = gradient from 90:10 to 75:25) to obtain 2.76 g of the compound 28 (7.49 mmol, yield 88%). The $^1$H-NMR data of the compound 28 obtained are shown below.

1H NMR (400 MHz, CDCl3, TMS as internal standard): δ 7.35 (br d, J = 7.3 Hz, 2 H), 7.24-7.09 (m, 3 H), 7.02 (br s, 2 H), 6.80 (br s, 1 H), 3.51-3.29 (m, 7 H), 2.79-2.65 (m, 1 H), 1.31-1.15 (m, 6 H), 1.11-0.96 (m, 6 H).

[Example 16]

<Synthesis of compound 29>

A compound 29 shown below was synthesized by a method described below according to the following reaction formula.

[Chem. 87]

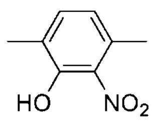

(Compound 29)

[Chem. 88]

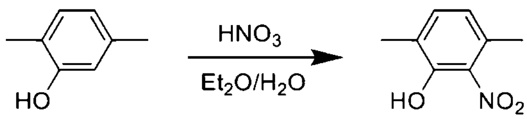


75.0 g of 2,5-dimethylphenol, 950 mL of diethyl ether, and 950 mL of water were put into an oven dried 3 L three-necked flask containing a heated and magnetic stirring bar in a nitrogen atmosphere and cooled in an ice bath while being stirred. Next, 79.8 g of fuming nitric acid (97%) was added dropwise thereto for one hour while the internal temperature was maintained at 4.8°C or lower. After the completion of the dropwise addition, the solution were stirred for 30 minutes, and the reaction solution was then extracted with diethyl ether. The combined organic layer was washed with water and brine once, then dried over magnesium sulfate, and concentrated with a rotary evaporator to obtain 96.7 g of a crude product. The crude product was purified by silica gel column chromatography (using a mixed solvent of hexane and ethyl acetate at 4:1) to obtain 25.8 g (yield 25%, red yellow solid) of the compound 29.

<Synthesis of compound 30>

A compound 30 shown below was synthesized by a method described below according to the following reaction formula.

[Chem. 89]

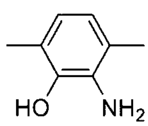

(Compound 30)

[Chem. 90]

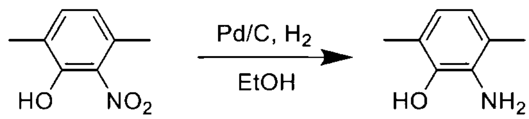


25.8 g of the compound 29 (155.2 mmol), 1014 ml of ethanol, and 136.6 mg of 10% Pd/C (55% water) were put into an oven dried 3 L three-necked flask containing a heated and magnetic stirring bar. A hydrogen balloon was connected to the flask, the atmosphere inside was substituted with hydrogen three times, the solution were stirred at room temperature for three hours, the hydrogen balloon was replaced, the inside was filled again with a hydrogen atmosphere, and the solution were stirred at room temperature for three hours. After that, the reaction solution was filtered with zeolite and washed with ethanol. The obtained filtrate was concentrated with a rotary evaporator to obtain 20.89 g of the crude product. A solid obtained by purifying the crude product by silica gel column chromatography (using a mixed solvent of dichloromethane and methanol as an eluent, dichloromethane: methanol = gradient from 1:0 to 19:1) and then concentrating the crude product was washed with toluene at 45°C and dried to obtain 16.76 g of the compound 30 with a yield 79%.

<Synthesis of compound 31>

A compound 31 shown below was synthesized by a method described below according to the following reaction formula.

[Chem. 91]

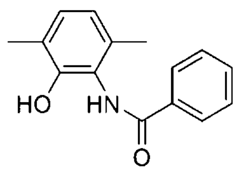

(Compound 31)

[Chem. 92]

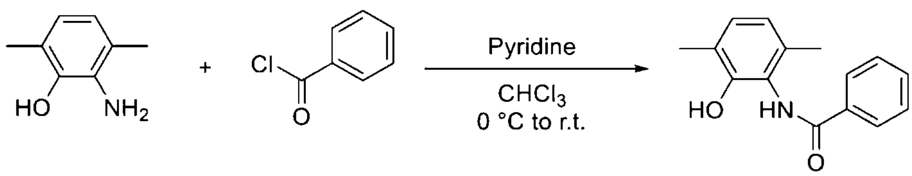


3.43 g of the compound 30 (25.0 mmol, 1 eq) and 100 mL of chloroform (dehydrated) were added into an oven dried 500 mL three-necked flask containing a heated and magnetic stirring bar in a nitrogen atmosphere. After that, 2.22 mL of pyridine (dehydrated, 27.5 mmol, 1.1 eq) was slowly added dropwise thereto at room temperature. After the reaction solution was cooled in an ice bath, 3.07 mL of benzoyl chloride (26.3 mmol, 1.05 eq) was slowly added dropwise thereto. After the end of the dropwise addition, the reaction solution was heated to room temperature and stirred for 4.5 hours. After the end of the reaction, the reaction solution was cooled again in an ice bath, 10 mL of methanol was added thereto, and the reaction solution was stirred. The reaction solution was filtered by adding water and dichloromethane thereto to obtain the compound 31 as a solid. The filtrate was concentrated with a rotary evaporator and filtered again by adding dichloromethane thereto to obtain the compound 31 as a solid. Furthermore, the same operation was repeated, and the obtained solid was collected to obtain 5.73 g (23.8 mmol, yield 95%) of the compound 31. The $^1$H-NMR data of the compound 31 obtained are shown below.

$^1$H NMR (400 MHz, $CDCl_3$, TMS as internal standard): δ 8.44 (s, 1 H), 7.95-7.90 (m, 2 H), 7.82 (br s, 1 H), 7.61 (tt, J = 7.4, 1.6 Hz, 1 H), 7.57-7.50 (m, 2 H), 7.00 (d, J = 7.6 Hz, 1 H), 6.72 (d, J = 7.6 Hz, 1H), 2.34 (s, 3 H), 2.30 (s, 3 H).

<Synthesis of compound 32>

A compound 32 shown below was synthesized by a method described below according to the following reaction formula.

[Chem. 93]

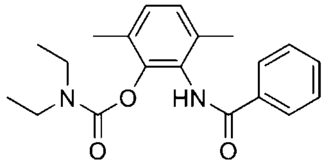

(Compound 32)

[Chem. 94]

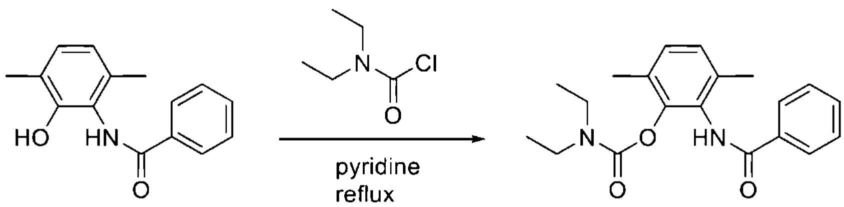


5.72 g of the compound 31 (23.7 mmol, 1 eq) and 130 mL of pyridine (dehydrated) were added into an oven dried 300 mL three-necked flask containing a heated and magnetic stirring bar in a nitrogen atmosphere. After that, 7.51 mL of diethylcarbamoyl chloride (59.3 mmol, 2.5 eq) was slowly added dropwise thereto at room temperature, and the solution were refluxed for 24 hours. After the end of the reaction, the reaction solution was cooled in an ice bath, 30 mL of water was slowly added thereto, and the reaction solution was stirred at room temperature. After that, the reaction solution was concentrated with a rotary evaporator, water and ethyl acetate were added thereto, and the reaction solution was extracted with ethyl acetate three times. The combined organic layer was washed with water three times and with brine once, dried over sodium sulfate, and then concentrated with the rotary evaporator. 8.17 g of the obtained crude product was purified by silica gel column chromatography (hexane and dichloromethane were used as an eluent, hexane: dichloromethane = gradient from 100:0 to 70:30) to obtain 4.03 g of the compound 32 (11.8 mmol, yield 50%). The $^1$H-NMR data of the compound 32 obtained are shown below.

$^1$H NMR (400 MHz, $CDCl_3$, TMS as internal standard): $\delta$ 7.93-7.85 (m, 2 H), 7.82 (br s, 1 H), 7.56-7.51 (m, 1 H), 7.49-7.42 (m, 2 H), 7.07 (q, J = 8.0 Hz, 2 H), 3.41-3.29 (m, 4 H), 2.28 (s, 3 H), 2.23 (s, 3 H) 1.16-0.99 (m, 6 H).

[Example 17]

<Synthesis of compound 33>

A compound 33 shown below was synthesized by a method described below according to the following reaction formula.

[Chem. 95]

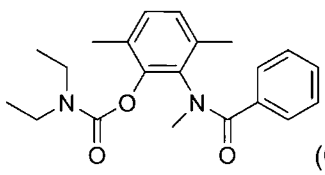

(Compound 33)

[Chem. 96]

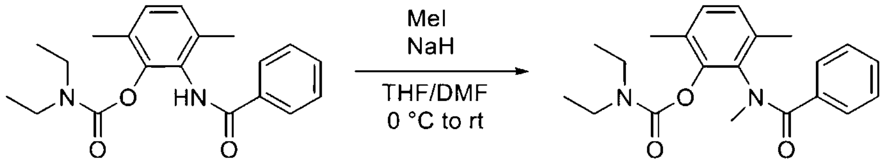


4.02 g of the compound 32 (11.8 mmol, 1 eq), 60 mL of tetrahydrofuran (dehydrated), and 6 mL of N,N'-dimethylformamide (dehydrated) were added into an oven dried 300 mL three-necked flask containing a heated and magnetic stirring bar in a nitrogen atmosphere. After the reaction solution was cooled in an ice bath, 0.67 g of sodium hydride (55%, liquid paraffin dispersion, 15.3 mmol, 1.3 eq) was slowly added thereto. After 30 minutes of stirring, 0.88 mL of iodomethane (14.2 mmol, 1.2 eq) was slowly added dropwise thereto. The reaction solution was slowly heated to room temperature and further stirred for five hours. After the end of the reaction, the reaction solution was cooled again in an ice bath, 10 mL of water was slowly added thereto, and the reaction solution was stirred at room temperature. Water and ethyl acetate were added thereto, and extraction was performed three times with ethyl acetate. The combined organic layer was washed with water three times and with brine once, dried over sodium sulfate, and then concentrated with a rotary evaporator. 4.45 g of the obtained crude product was purified by silica gel column chromatography (eluent, hexane: ethyl acetate = gradient from 90:0 to 70:30), and was recrystallized with methanol to obtain 3.24 g of the compound 33 (9.14 mmol, yield 78%). The $^1$H-NMR data of the compound 33 obtained are shown below. (Two kinds of rotamers are generated and each denoted as a major component or a minor component. The ratio therebetween is major:minor = 92:8.)

$^1$H NMR (400 MHz, $CDCl_3$, TMS as internal standard):

Major component: δ 7.35 (br s, 2 H), 7.25-7.20 (m, 1 H), 7.17-7.10 (m, 2 H), 6.99 (br d, J = 7.8 Hz, 1 H), 6.87 (br d, J = 7.8 Hz, 1 H), 3.64-3.22 (m, 7 H), 2.26-1.99 (m, 6 H), 1.33-1.15 (m, 6 H).
Minor component: δ 7.55-7.50 (m, 2 H), 7.46-7.41 (m, 3 H), 7.17-7.10 (m, 1 H), 7.07 (br d, J =8.0 Hz, 1 H), 3.64-3.22 (m, 4 H), 3.14 (s, 3 H), 2.30 (s, 3 H), 2.26-1.99 (m, 3 H), 1.33-1.15 (m, 6 H).

## Claims

**1.** A carbamate compound represented by the following formula (0):

[Chem. 1]

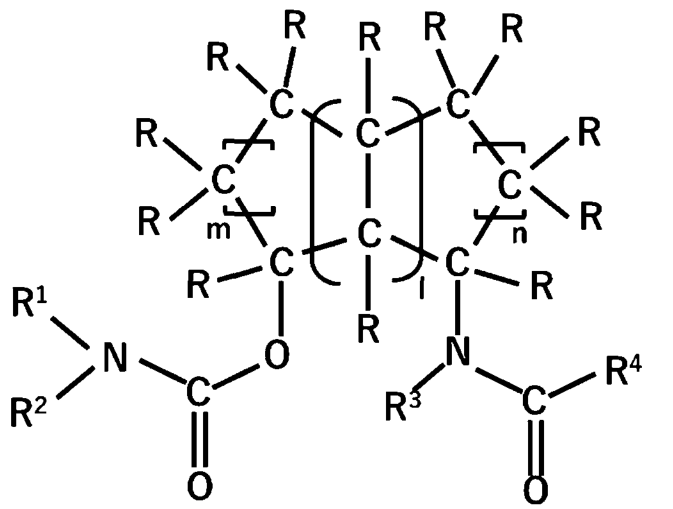

(0)

wherein

l is 0 or 1,
m is an integer of 1 to 4,
n is an integer of 1 to 4,

$R^1$ and $R^2$ are each a substituent having a "$R^{10}$-$CR_2$-" structure,
$R^3$ is a hydrogen atom or a substituent having a "$R^{10}$-$CR_2$-" structure,
$R^4$ is a substituent selected from a substituent having a "$R^{10}$-$CR_2$-" structure, a substituent having a "$R^{10}$-$At^{16}$-" structure, and a substituent having a "$R^{10}_2$-$At^{15}$-" structure,
$At^{15}$ is a Group 15 atom in the periodic table, and $At^{16}$ is a Group 16 atom in the periodic table,
R and $R^{10}$ are each a group containing an atom selected from carbon, hydrogen, and elements in Groups 15, 16, and 17 of the periodic table, which has 0 to 17 carbon atoms and 0 to 4 atoms of the elements in Groups 15, 16, and 17 of the periodic table,
$R^1$ to $R^4$ and R may bond to each other to form a monocyclic or polycyclic ring, and
a plurality of R and $R^{10}$ may bond to each other to form a monocyclic or polycyclic ring or form a multiple bond.

**2.** The carbamate compound according to Claim 1, wherein the compound is represented by the following formula (1):

[Chem. 2]

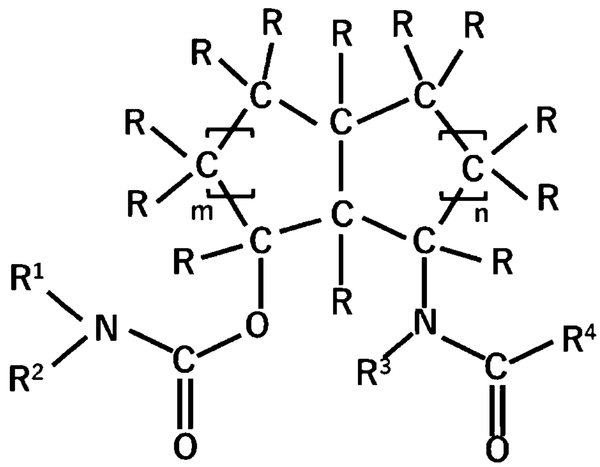

(1)

wherein n is an integer of 2 to 4, and m, $R^1$, $R^2$, $R^3$, $R^4$, and
R have the same meaning as m, $R^1$, $R^2$, $R^3$, $R^4$, and R in the formula (0).

**3.** The carbamate compound according to Claim 1 or 2, wherein the $R^3$ is a substituent having a "$R^{10}$-$CR_2$-" structure.

**4.** The carbamate compound according to Claim 1 or 2, wherein the $R^4$ is a substituent having a "$R^{10}$-$CR_2$-" structure.

**5.** The carbamate compound according to Claim 1 or 2, wherein the $At^{15}$ is a nitrogen atom.

**6.** The carbamate compound according to Claim 1 or 2, wherein the $At^{16}$ is an oxygen atom.

**INTERNATIONAL SEARCH REPORT**

International application No. **PCT/JP2024/013473**

**A. CLASSIFICATION OF SUBJECT MATTER**

***C07C 271/44***(2006.01)i; ***C07C 275/32***(2006.01)i; ***C07D 295/205***(2006.01)i
FI: C07C271/44 CSP; C07C275/32; C07D295/205

According to International Patent Classification (IPC) or to both national classification and IPC

**B. FIELDS SEARCHED**

Minimum documentation searched (classification system followed by classification symbols)

C07C271/44; C07C275/32; C07D295/205

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | JP 8-201957 A (KONICA CORP.) 09 August 1996 (1996-08-09) paragraphs [0040]-[0045] | 1-6 |
| X | EP 0704757 A1 (KONICA CORPORATION) 03 April 1996 (1996-04-03) pages 11-16 | 1-6 |
| X | JP 5-107708 A (FUJI PHOTO FILM CO., LTD.) 30 April 1993 (1993-04-30) page 24 | 1-6 |
| X | US 4861701 A (EASTMAN KODAK COMPANY) 29 August 1989 (1989-08-29) columns 55-56 | 1-6 |
| X | JP 1-203351 A (MITSUI PETROCHEM IND. LTD.) 16 August 1989 (1989-08-16) table 1 | 1-6 |
| X | JP 56-154449 A (KUMIAI CHEMICAL INDUSTRY CO., LTD.) 30 November 1981 (1981-11-30) table 1 | 1-6 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **05 June 2024** | **25 June 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

INTERNATIONAL SEARCH REPORT

International application No.
PCT/JP2024/013473

C. DOCUMENTS CONSIDERED TO BE RELEVANT

| Category* | Citation of document, with indication, where appropriate, of the relevant passages | Relevant to claim No. |
|---|---|---|
| X | WO 2015/011164 A1 (LES LABORATOIRES SERVIER) 29 January 2015 (2015-01-29) pages 248, 260 | 1-2, 4-6 |
| X | EP 0846982 A2 (FUJI PHOTO FILM CO., LTD.) 10 June 1998 (1998-06-10) page 83 | 1-2, 4-6 |
| X | BERA, S. S. et al., Carbamates: A Directing Group for Selective C-H Amidation and Alkylation under Cp*Co(III) Catalysis, Organic Letters, 2020, vol. 22, no. 7, pages 2615-2620, DOI:10.1021/acs.orglett.0c00589 scheme 5 | 1-2, 4-6 |
| X | WANG, H. et al., Overcoming the Limitations of C-H Activation with Strongly Coordinating N-Heterocycles by Cobalt Catalysis, Angewandte Chemie International Edition, 2016, vol. 55, no. 35, pages 10386-10390, DOI:10.1002/anie.201603260 scheme 3 | 1-2, 4-6 |
| X | RAIFORD, L. C. et al., Migration of the Carbamyl Radical in 2-Aminophenol Derivatives, The Journal of Organic Chemistry, 1940, vol. 05, no. 3, pages 300-312, DOI:10.1021/jo01209a012 page 303 | 1-2, 4-6 |
| X | Database Registry, 2020, RN 2425032-79-5, RN 1049973-85-4, RN 1049973-78-5, RN 1049973-72-9, RN 1049971-53-0, RN 1049971-43-8, RN 1049715-55-0, RN 1049715-00-5, RN 1049714-27-3, RN 1049694-82-7, RN 1049694-54-3, RN 792144-52-6, RN 774495-45-3, RN 773805-96-2, RN 773012-18-3, RN 770666-50-7, RN 767586-79-8, RN 761358-18-3, Retrieved from STN international [online], retrieved on 4 June 2024 sections of RN, CN | 1-2, 4-6 |
| X | Database Registry, 2004, RN 756775-41-4, RN 741631-58-3, RN 739325-31-6, RN 738540-24-4, RN 727645-04-7, RN 689213-53-4, RN 664968-36-9, RN 428839-70-7, RN 93392-59-7, RN 93392-57-5, RN 93392-55-3, RN 93392-53-1, RN 93392-47-3, RN 93392-44-0, RN 93392-42-8, RN 93392-38-2, RN 93392-18-8, Retrieved from STN international [online], retrieved on 4 June 2024 sections of RN, CN | 1-2, 4-6 |
| X | WO 98/27055 A1 (TEVA PHARMACEUTICAL INDUSTRIES, LTD.) 25 June 1998 (1998-06-25) table 1 | 1, 3-6 |
| A | CN 108570119 A (BEIJING LIHE ZHIXIN TECHNOLOGY CO., LTD.) 25 September 2018 (2018-09-25) entire text | 1-6 |
| A | CN 108570120 A (BEIJING LIHE ZHIXIN TECHNOLOGY CO., LTD.) 25 September 2018 (2018-09-25) entire text | 1-6 |
| A | WO 2011/106500 A1 (DOW GLOBAL TECHNOLOGIES LLC) 01 September 2011 (2011-09-01) entire text | 1-6 |

Form PCT/ISA/210 (second sheet) (July 2022)

**INTERNATIONAL SEARCH REPORT**
**Information on patent family members**

International application No.
**PCT/JP2024/013473**

| Patent document cited in search report | Publication date (day/month/year) | Patent family member(s) | Publication date (day/month/year) |
|---|---|---|---|
| JP 8-201957 A | 09 August 1996 | (Family: none) | |
| EP 0704757 A1 | 03 April 1996 | JP 8-328195 A<br>US 5616446 A | |
| JP 5-107708 A | 30 April 1993 | (Family: none) | |
| US 4861701 A | 29 August 1989 | (Family: none) | |
| JP 1-203351 A | 16 August 1989 | (Family: none) | |
| JP 56-154449 A | 30 November 1981 | (Family: none) | |
| WO 2015/011164 A1 | 29 January 2015 | JP 2016-525529 A<br>US 2016/0194304 A1<br>EP 3024826 A1<br>CN 105431422 A<br>KR 10-2016-0033224 A | |
| EP 0846982 A2 | 10 June 1998 | JP 10-207030 A<br>US 6051359 A | |
| WO 98/27055 A1 | 25 June 1998 | JP 2001-506269 A<br>US 2002/0188020 A1<br>EP 966435 A1 | |
| CN 108570119 A | 25 September 2018 | (Family: none) | |
| CN 108570120 A | 25 September 2018 | (Family: none) | |
| WO 2011/106500 A1 | 01 September 2011 | JP 2013-521341 A<br>US 2012/0316299 A1<br>EP 2539379 A1<br>CN 102918068 A<br>KR 10-2013-0020771 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2005226076 A
- JP 2000516987 A
- JP 2002542347 A
- JP 2005517746 A
- JP 2011529888 A
- JP 2014500390 A
- WO 2016184884 A
- US 10005859 B
- US 10836847 B